# EUROPEAN PATENT SPECIFICATION

(11) **EP 3 778 582 B1**
(45) Date of publication and mention of the grant of the patent: **20.07.2022**
(21) Application number: 19860054.6
(22) Date of filing: 11.09.2019
(51) Int. Cl.: C07D 307/91, H01L 51/00, C09K 11/00, C07F 7/08, C09K 11/06

(54) **NOVEL COMPOUND, COATING COMPOSITION COMPRISING THE SAME AND ORGANIC LIGHT EMITTING DEVICE COMPRISING THE SAME**
NEUE VERBINDUNG, BESCHICHTUNGSZUSAMMENSETZUNG, DIE DIESE UMFASST UND ORGANISCHE LEUCHTDIODE, DIE DIESE UMFASST
NOUVEAU COMPOSÉ, COMPOSITION DE REVÊTEMENT LE COMPRENANT ET DIODE ÉLECTROLUMINESCENTE ORGANIQUE COMPRENANT CELUI-CI

(30) Priority: 14.09.2018 KR 20180110277
(43) Date of publication of application: 17.02.2021
(73) Proprietor: LG CHEM, LTD., Yeongdeungpo-gu, Seoul 07336 (KR)
(72) Inventor: KIM, Yongwook, Daejeon 34122 (KR); BAE, Jaesoon, Daejeon 34122 (KR); LEE, Jaechol, Daejeon 34122 (KR); LEE, Kilsun, Daejeon 34122 (KR); KIM, Young Kwang, Daejeon 34122 (KR); BAEK, Leehyeon, Daejeon 34122 (KR); PARK, Hyungil, Daejeon 34122 (KR)
(74) Representative: Hoffmann Eitle
(86) International application number: PCT/KR2019/011851
(87) International publication number: WO 2020/055177

(56) References cited:
- KR-A- 20170 039 020
- KR-A- 20170 044 001
- KR-A- 20170 044 001
- KR-A- 20180 037 695
- KR-A- 20180 037 717
- KR-A- 20180 077 887

## Description

### [TECHNICAL FIELD]

### CROSS-REFERENCE TO RELATED APPLICATION(S)

This application claims the benefit of the filing dates of Korean Patent Application No. 10-2018-0110277 filed with Korean Intellectual Property Office on September 14, 2018, and Korean Patent Application No. 10-2019-0112756 filed with Korean Intellectual Property Office on September 11, 2019.

The present invention relates to a novel compound, a coating composition including the same, and an organic light emitting device including the same.

### [BACKGROUND ART]

In general, an organic light emitting phenomenon refers to a phenomenon where electrical energy is converted into light energy by using an organic material. The organic light emitting device using the organic light emitting phenomenon has characteristics such as a wide viewing angle, excellent contrast, a fast response time, and excellent luminance, driving voltage, and response speed, and thus many studies have proceeded thereon.

The organic light emitting device generally has a structure which includes an anode, a cathode, and an organic material layer interposed between the anode and the cathode.

The organic material layer frequently has a multilayered structure that includes different materials in order to enhance efficiency and stability of the organic light emitting device, and for example, the organic material layer may be formed of a hole injection layer, a hole transport layer, a light emitting layer, an electron transport layer, an electron injection layer, and the like.

In the structure of the organic light emitting device, if a voltage is applied between two electrodes, the holes are injected from an anode into the organic material layer and the electrons are injected from the cathode into the organic material layer, and when the injected holes and electrons meet each other, an exciton is formed, and light is emitted when the exciton falls to a ground state again.

There is a continuing need for the development of new materials for the organic materials used in the organic light emitting devices as described above.

Patent Literature 0002 describes an organic light-emitting device comprising a light-emittting layer including an amine derivative.

### [Prior Art Literature]

### [Patent Literature]

(Patent Literature 0001) Korean Patent Laid-open Publication No. 10-2000-0051826
(Patent Literature 0002) Korean Patent Laid-open publication No. 2017-00440001

### [DETAILED DESCRIPTION OF THE INVENTION]

### [TECHNICAL PROBLEM]

It is an object of the present invention to provide a novel compound, a coating composition including the same, and an organic light emitting device including the same.

### [TECHNICAL SOLUTION]

In one aspect of the invention, there is provided a compound represented by the following Chemical Formula 1: 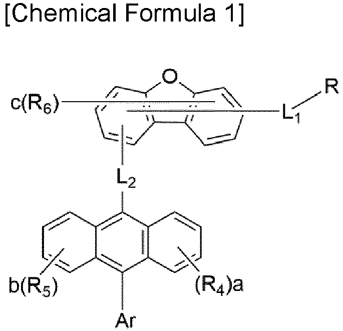 wherein, in Chemical Formula 1,
R is Si(R₁)(R₂)(R₃),
R₁ to R₃ are each independently hydrogen; a substituted or unsubstituted C₁₋₆₀ alkyl; a substituted or unsubstituted C₆₋₆₀ aryl; a (tri(C₁₋₆₀ alkyl)silyl)-(C₁₋₁₀ alkylene)-; or a (th(C₆₋₆₀ aryl)silyl)-(C₁₋₁₀ alkylene)-, or R₁ and R₂ are linked together to form a ring, provided that the case wherein R₁ to R₃ are all a substituted or unsubstituted C₁₋₆₀ alkyl is excluded,
a to c are each independently an integer of 0 to 4,
R₄ to R₆ are each independently hydrogen; deuterium; a halogen; a cyano; a substituted or unsubstituted C₁₋₆₀ alkyl; a substituted or unsubstituted C₁₋₆₀ alkoxy; a substituted or unsubstituted C₁₋₆₀ thioalkyl; a substituted or unsubstituted C₃₋₆₀ cycloalkyl; a substituted or unsubstituted C₆₋₆₀ aryl; or a substituted or unsubstituted C₅₋₆₀ heteroaryl containing one or more heteroatoms selected from the group consisting of N, O, and S,
L₁ is a single bond; or phenylene,
L₂ is a single bond; or a substituted or unsubstituted C₆₋₆₀ arylene, and
Ar is a substituted or unsubstituted C₆₋₆₀ aryl; or substituted or unsubstituted C₅₋₆₀ heteroaryl containing one or more heteroatoms selected from the group consisting of N, O, and S.

In one aspect of the invention, there is provided a coating composition including the compound represented by Chemical Formula 1.

In another aspect of the invention, there is provided an organic light emitting device including: a first electrode; a second electrode provided opposite to the first electrode; and one or more organic material layers provided between the first electrode and the second electrode, wherein one or more of the organic material layers include the compound represented by Chemical Formula 1.

### [ADVANTAGEOUS EFFECTS]

The compound represented by Chemical Formula 1 described above can be used as a material of an organic material layer of an organic light emitting device, and may improve the efficiency, and achieve a low driving voltage and/or improve lifetime characteristics in the organic light emitting device. In particular, the compound represented by Chemical Formula 1 described above can be used as a hole injection material, a hole transport material, a hole injection and transport material, a light emitting material, an electron transport material, or an electron injection material.

### [BRIEF DESCRIPTION OF DRAWINGS]

FIG. 1 shows an example of an organic light emitting device including a substrate 1, an anode 2, a light emitting layer 3, and a cathode 4.
FIG. 2 shows an example of an organic light emitting device including a substrate 1, an anode 2, a hole injection layer 5, a hole transport layer 6, a light emitting layer 7, an electron transport layer 8, and a cathode 4.

### [DETAILED DESCRIPTION OF THE EMBODIMENTS]

Hereinafter, embodiments of the present invention will be described in more detail to facilitate understanding of the present invention.

One embodiment of the present invention provides a compound represented by Chemical Formula 1.

As used herein, the notation 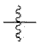 or 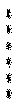 means a bond linked to another substituent group.

In the present invention, the term "substituted or unsubstituted" means being unsubstituted or substituted with one or more substituents selected from the group consisting of deuterium; a halogen group; a nitrile group; a nitro group; a hydroxy group; a carbonyl group; an ester group; an imide group; an amino group; a phosphine oxide group; an alkoxy group; an aryloxy group; an alkylthioxy group; an arylthioxy group; an alkylsulfoxy group; an arylsulfoxy group; a silyl group; a boron group; an alkyl group; a cycloalkyl group; an alkenyl group; an aryl group; an aralkyl group; an aralkenyl group; an alkylaryl group; an alkylamine group; an aralkylamine group; a heteroarylamine group; an arylamine group; an arylphosphine group; and a heterocyclic group containing at least one of N, O, and S atoms, or being unsubstituted or substituted with a substituent to which two or more substituents are linked among the substituents exemplified above.

For example, "the substituent to which two or more substituents are linked" may be a biphenyl group.

That is, the biphenyl group may also be an aryl group, and may be interpreted as a substituent to which two phenyl groups are linked.

In the present specification, the number of carbon atoms of a carbonyl group is not particularly limited, but is preferably 1 to 40.

Specifically, the carbonyl group may be a compound having the following structural formulas, but is not limited thereto.

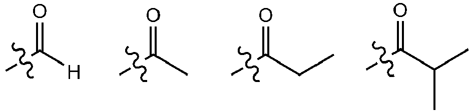

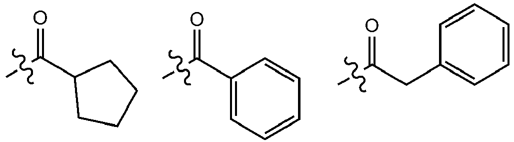

In the present specification, an ester group may have a structure in which oxygen of the ester group may be substituted by a straight-chain, branched-chain, or cyclic alkyl group having 1 to 25 carbon atoms, or an aryl group having 6 to 25 carbon atoms.

Specifically, the ester group may be a compound having the following structural formulas, but is not limited thereto.

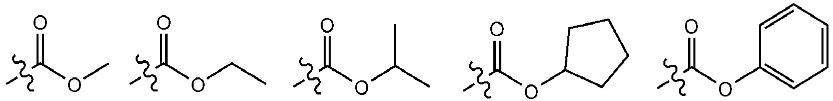

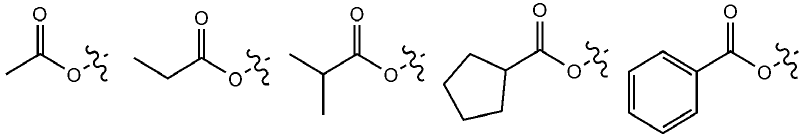

In the present specification, the number of carbon atoms of an imide group is not particularly limited, but is preferably 1 to 25.

Specifically, the imide group may be a compound having the following structural formulas, but is not limited thereto.

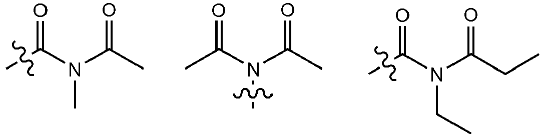

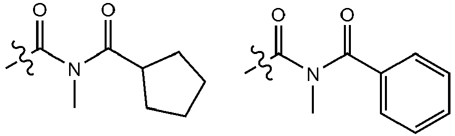

In the present specification, a silyl group specifically includes a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, and the like, but is not limited thereto.

In the present specification, a boron group specifically includes a trimethylboron group, a triethylboron group, a t-butyldimethylboron group, a triphenylboron group, and a phenylboron group, but is not limited thereto.

In the present specification, examples of a halogen group include fluorine, chlorine, bromine, and iodine.

In the present specification, the alkyl group may be straight-chain or branched-chain, and the number of carbon atoms thereof is not particularly limited, but is preferably 1 to 40.

According to one embodiment, the number of carbon atoms of the alkyl group is 1 to 20.

According to another embodiment, the number of carbon atoms of the alkyl group is 1 to 10.

According to another embodiment, the number of carbon atoms of the alkyl group is 1 to 6.

Specific examples of the alkyl group include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 4-methylhexyl, 5-methylhexyl, and the like, but are not limited thereto.

In the present specification, the alkenyl group may be straight-chain or branched-chain, and the number of carbon atoms thereof is not particularly limited, but is preferably 2 to 40.

According to one embodiment, the number of carbon atoms of the alkenyl group is 2 to 20.

According to another embodiment, the number of carbon atoms of the alkenyl group is 2 to 10. According to still another embodiment, the number of carbon atoms of the alkenyl group is 2 to 6.

Specific examples thereof include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a stilbenyl group, a styrenyl group, and the like, but are not limited thereto.

In the present specification, a cycloalkyl group is not particularly limited, but the number of carbon atoms thereof is preferably 3 to 60. According to one embodiment, the number of carbon atoms of the cycloalkyl group is 3 to 30.

According to another embodiment, the number of carbon atoms of the cycloalkyl group is 3 to 20.

According to still another embodiment, the number of carbon atoms of the cycloalkyl group is 3 to 6.

Specific examples thereof include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, and the like, but are not limited thereto.

In the present specification, an aryl group is not particularly limited, but preferably has 6 to 60 carbon atoms, and it may be a monocyclic aryl group or a polycyclic aryl group.

According to one embodiment, the number of carbon atoms of the aryl group is 6 to 30.

According to one embodiment, the number of carbon atoms of the aryl group is 6 to 20.

The aryl group may be a phenyl group, a biphenyl group, a terphenyl group, or the like as the monocyclic aryl group, but is not limited thereto.

The polycyclic aryl group includes a naphthyl group, an anthracenyl group, a phenanthryl group, a pyrenyl group, a perylenyl group, a chrysenyl group, a fluorenyl group, or the like, but is not limited thereto.

In the present specification, a fluorenyl group may be substituted, and two substituent groups may be linked to each other to form a spiro structure.

In the case where the fluorenyl group is substituted, 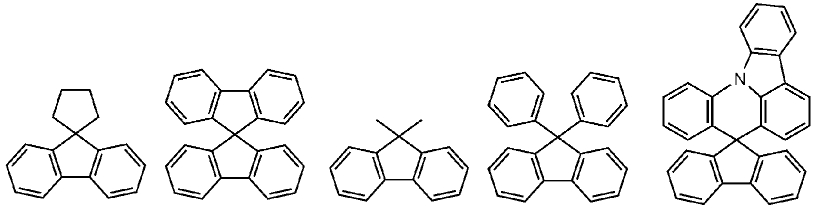 and the like can be formed. However, the structure is not limited thereto.

In the present specification, a heterocyclic group is a heterocyclic group including one or more of O, N, P, Si, and S as a heteroatom, and the number of carbon atoms thereof is not particularly limited, but is preferably 2 to 60.

Examples of the heterocyclic group include a thiophene group, a furan group, a pyrrole group, an imidazole group, a thiazole group, an oxazole group, an oxadiazole group, a triazole group, a pyridyl group, a bipyridyl group, a pyrimidyl group, a triazine group, an acridyl group, a pyridazine group, a pyrazinyl group, a quinolinyl group, a quinazoline group, a quinoxalinyl group, a phthalazinyl group, a pyridopyrimidinyl group, a pyridopyrazinyl group, a pyrazinopyrazinyl group, an isoquinoline group, an indole group, a carbazole group, a benzoxazole group, a benzimidazole group, a benzothiazole group, a benzocarbazole group, a benzothiophene group, a dibenzothiophene group, a benzofuranyl group, a phenanthroline group, an isoxazolyl group, a thiadiazolyl group, a phenothiazinyl group, a dibenzofuranyl group, and the like, but are not limited thereto.

In the present specification, the aryl group in the aralkyl group, the aralkenyl group, the alkylaryl group, and the arylamine group is the same as the aforementioned examples of the aryl group.

In the present specification, the alkyl group in the aralkyl group, the alkylaryl group, and the alkylamine group is the same as the aforementioned examples of the alkyl group.

In the present specification, the heteroaryl in the heteroarylamine group can be applied to the aforementioned description of the heterocyclic group.

In the present specification, the alkenyl group in the aralkenyl group is the same as the aforementioned examples of the alkenyl group.

In the present specification, the aforementioned description of the aryl group may be applied except that the arylene is a divalent group.

In the present specification, the aforementioned description of the heterocyclic group can be applied except that the heteroarylene is a divalent group.

In the present specification, the aforementioned description of the aryl group or cycloalkyl group can be applied except that the hydrocarbon ring is not a monovalent group but is formed by combining two substituent groups.

In the present specification, the aforementioned description of the heterocyclic group can be applied, except that the hetero ring is not a monovalent group but is formed by combining two substituent groups.

Preferably, Chemical Formula 1 may be the following Chemical Formula 1-1 or 1-2: 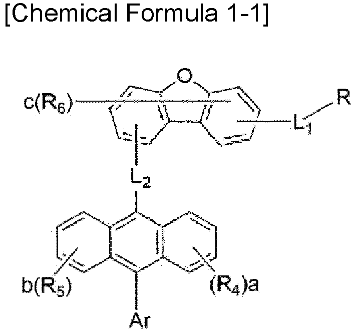 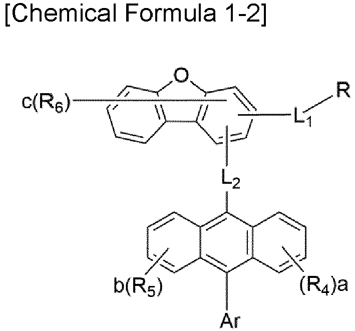 wherein, in Chemical Formula 1-1 or 1-2,
R, a to c, R₄ to R₆, L₁, L₂, and Ar are as defined above.

In Chemical Formula 1, preferably, a to c may be all 0.

Preferably, R₁ to R₃ are each independently methyl; i-propyl; t-butyl; phenyl; trimethylsilyl-ethylene; or triphenylsilyl-ethylene, or R₁ and R₂ may be linked together to form a cyclopentyl; cyclohexyl; or 2,3-dihydro-1H-indenyl ring.

Preferably, at least one of R₁ to R₃ may be phenyl.

Preferably L₂ may be a single bond or phenylene.

Further, preferably, Ar may be any one selected from the group consisting of the following.

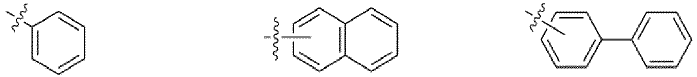

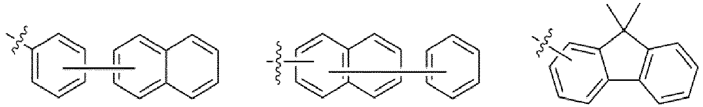

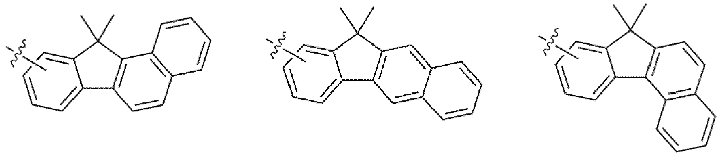

For example, the aforementioned compound may be selected from the group consisting of the following compounds.

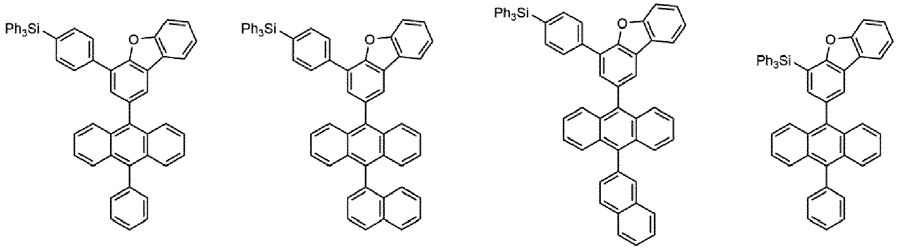

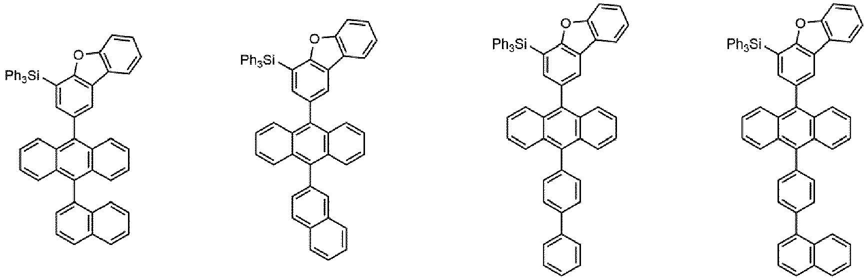

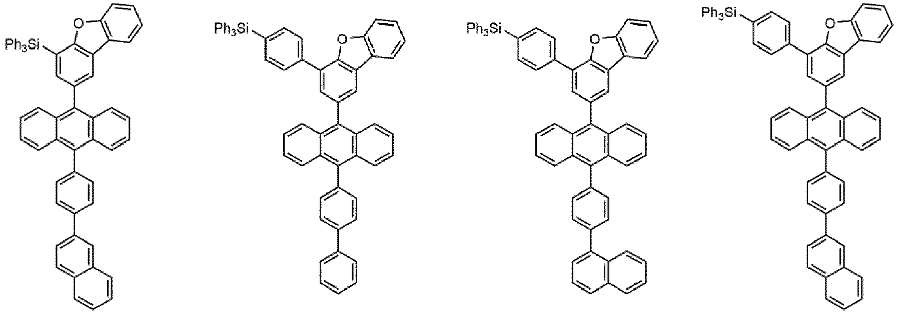

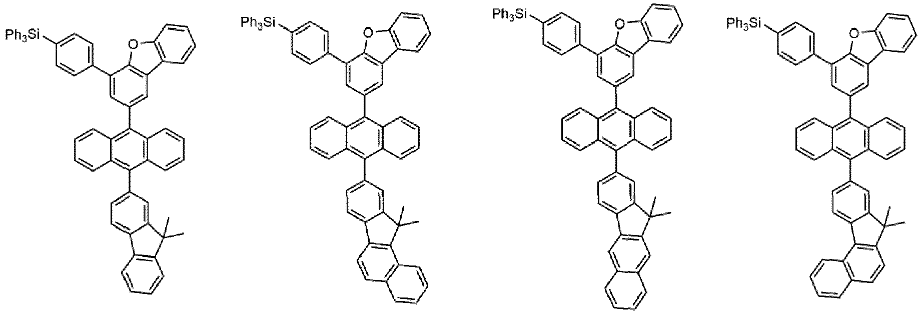

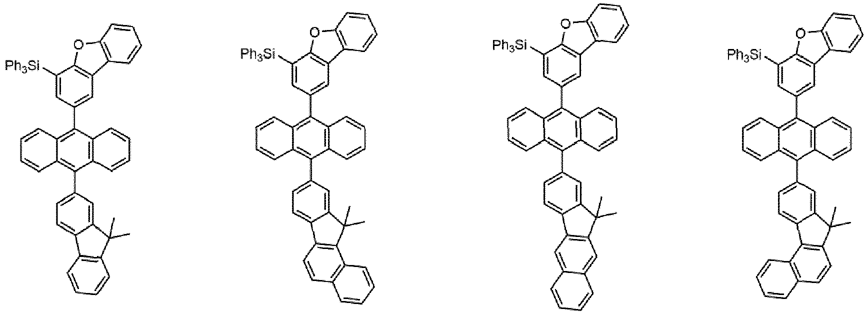

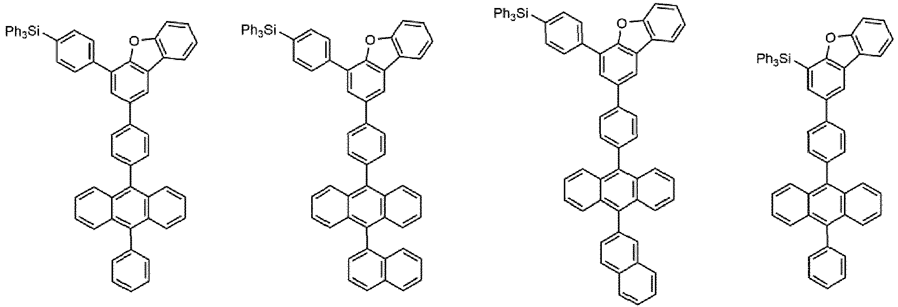

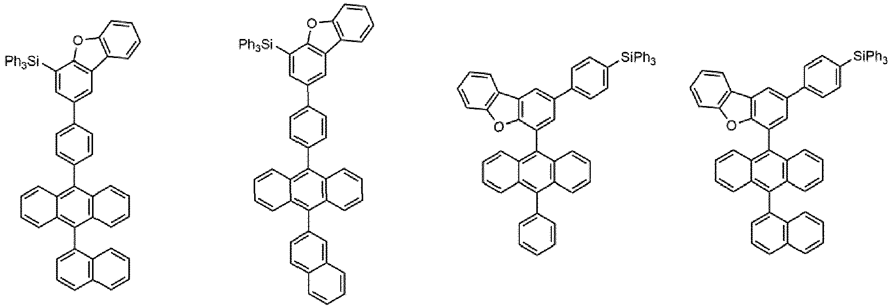

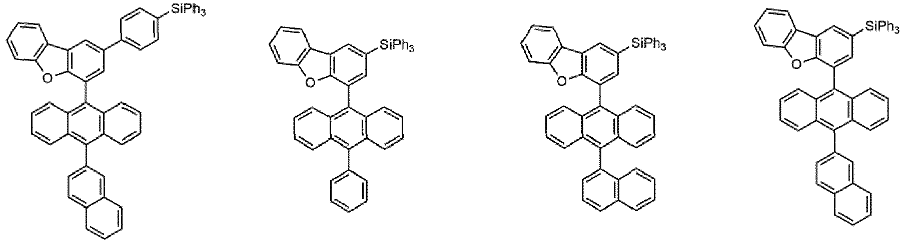

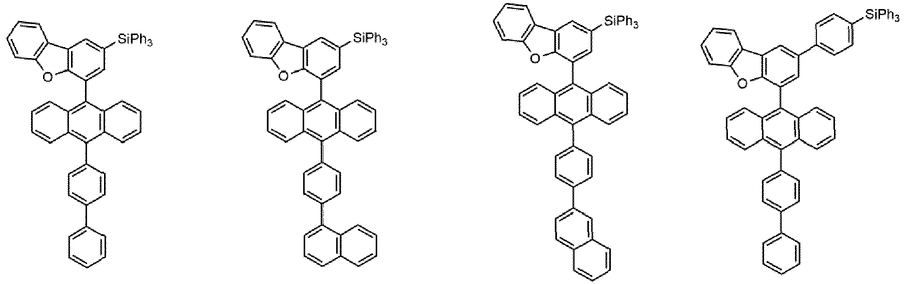

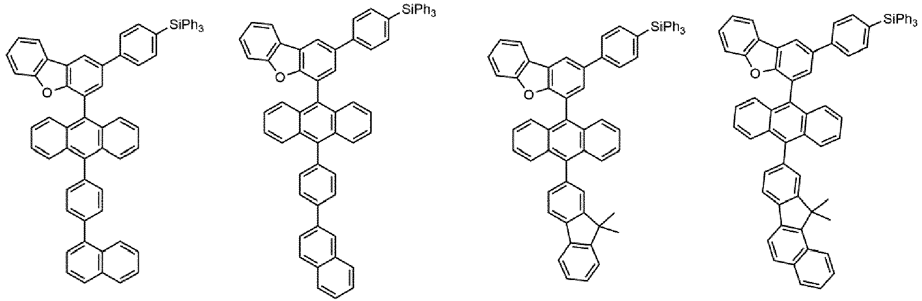

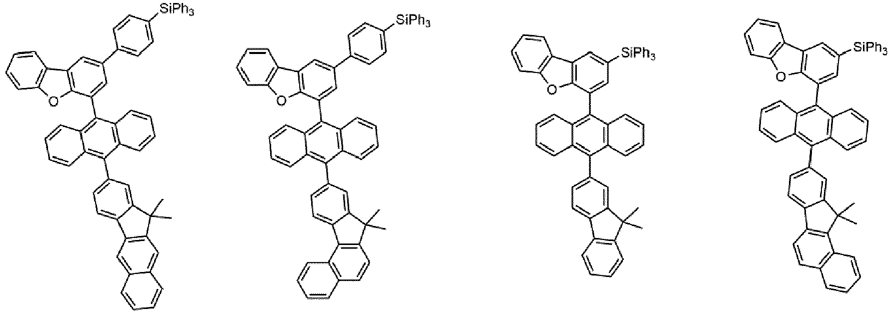

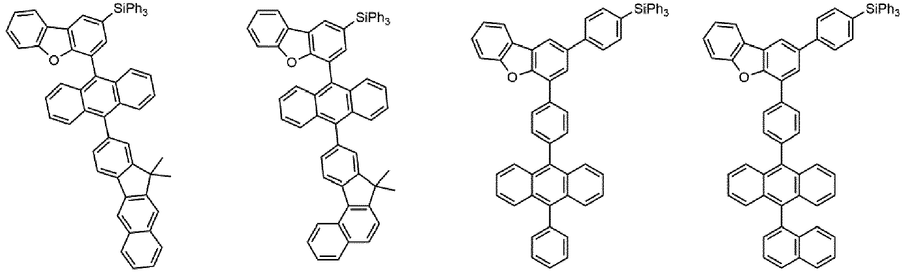

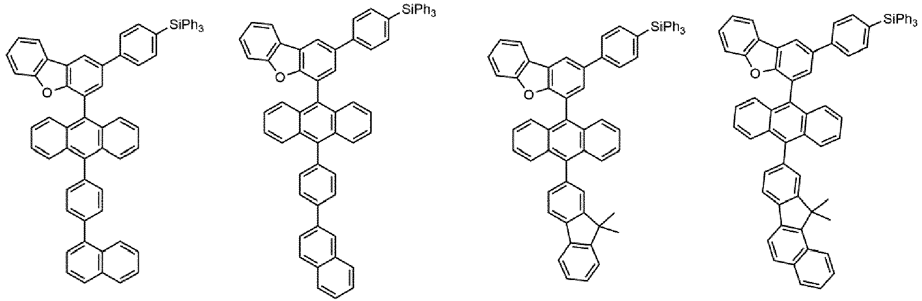

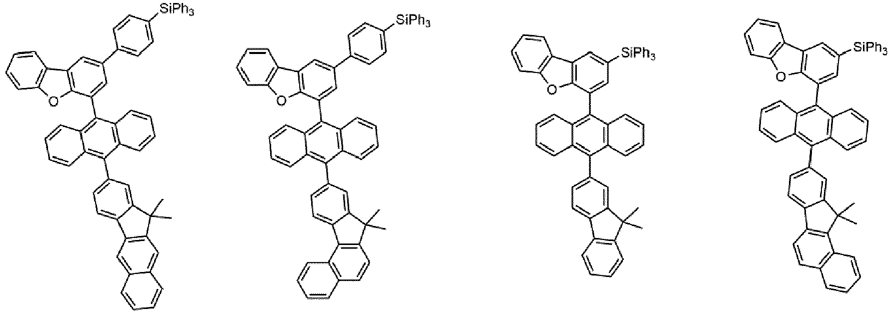

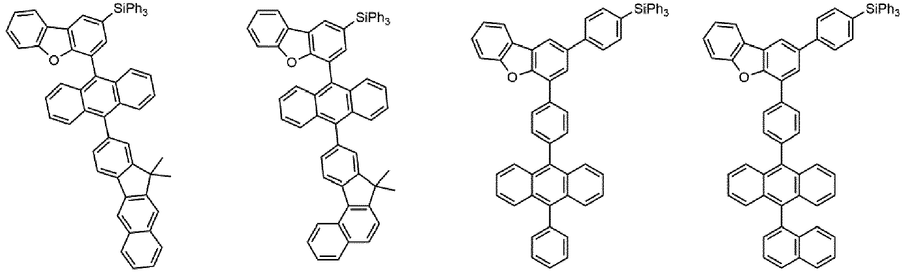

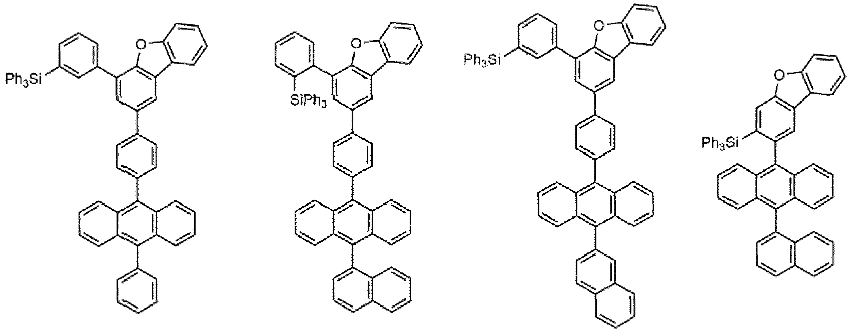

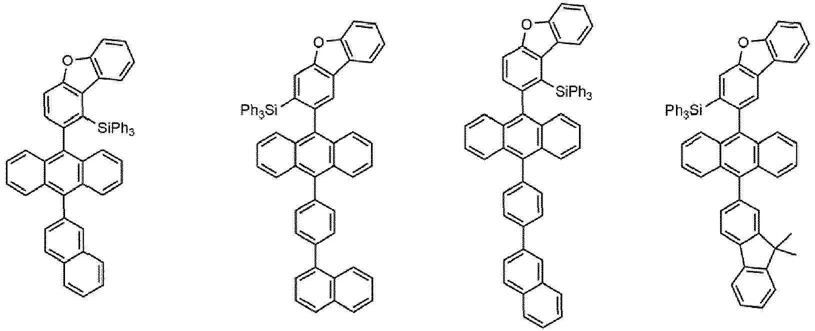

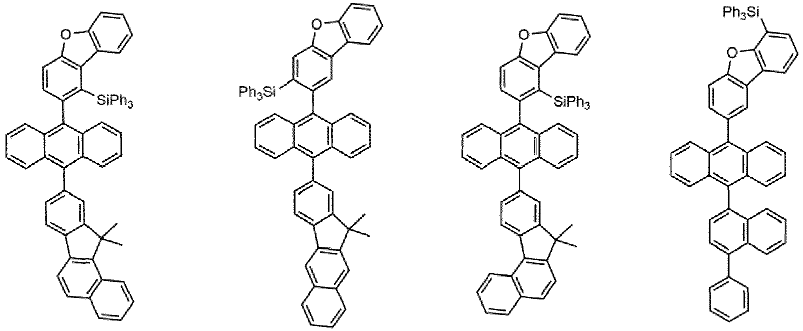

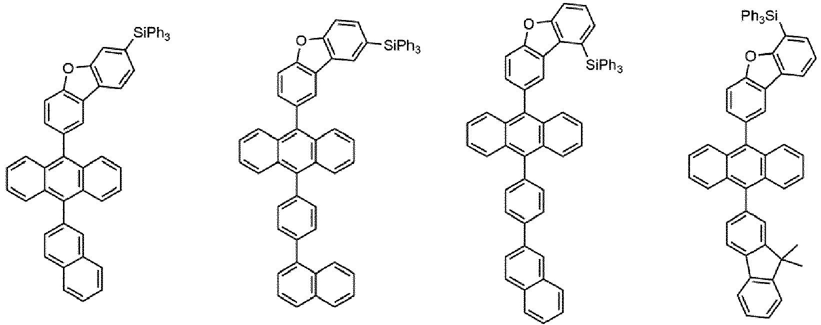

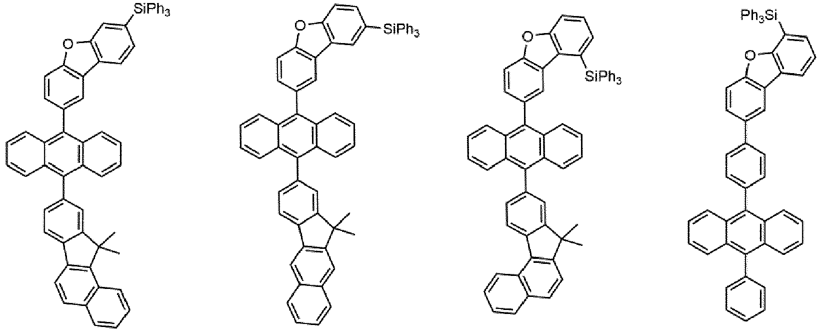

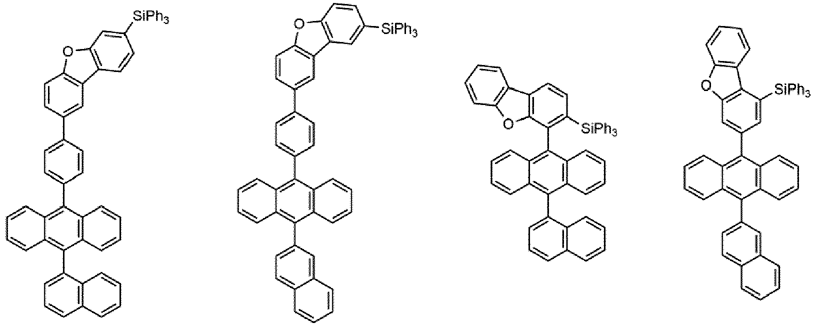

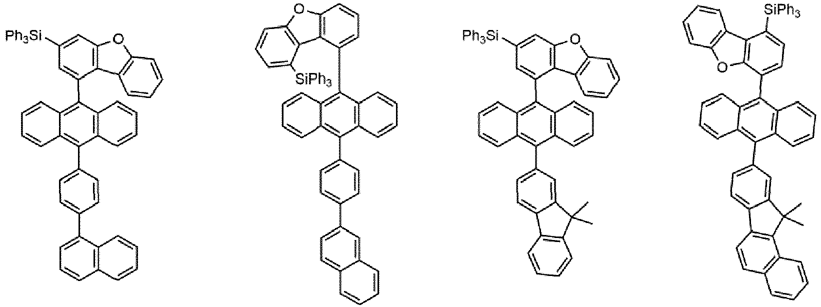

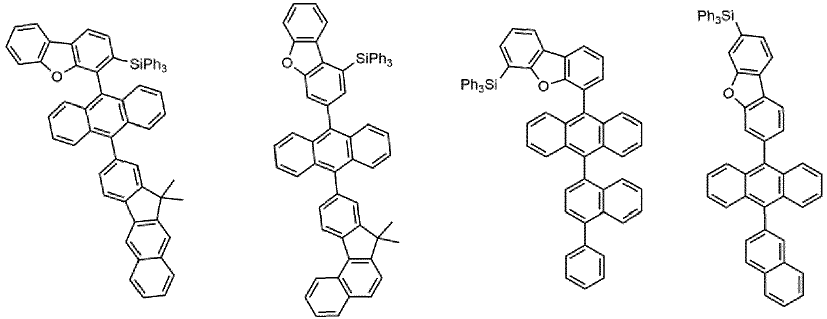

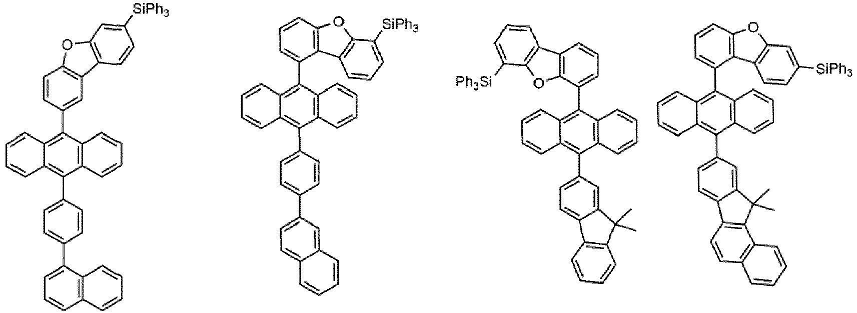

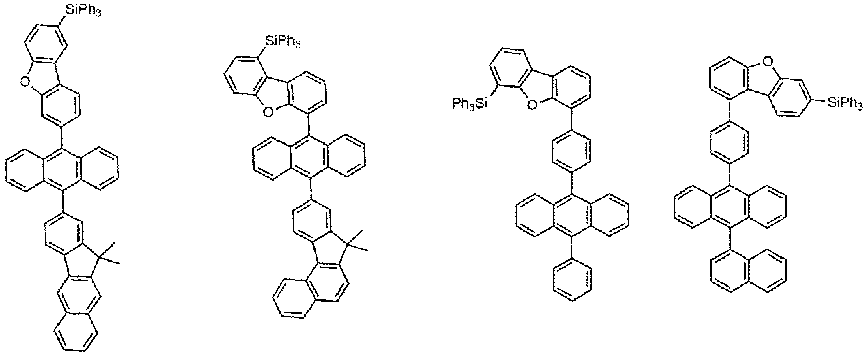

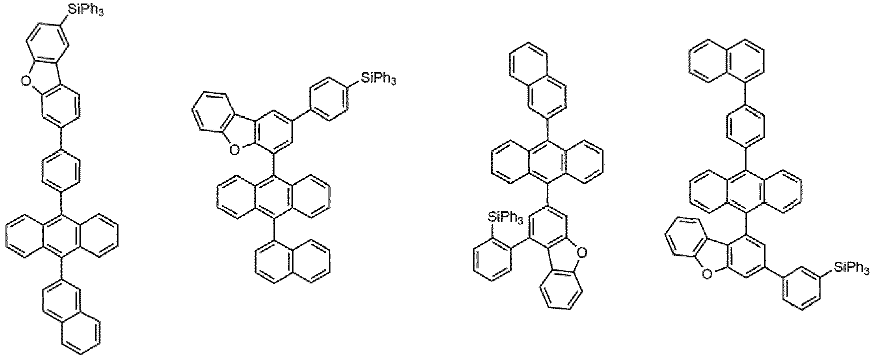

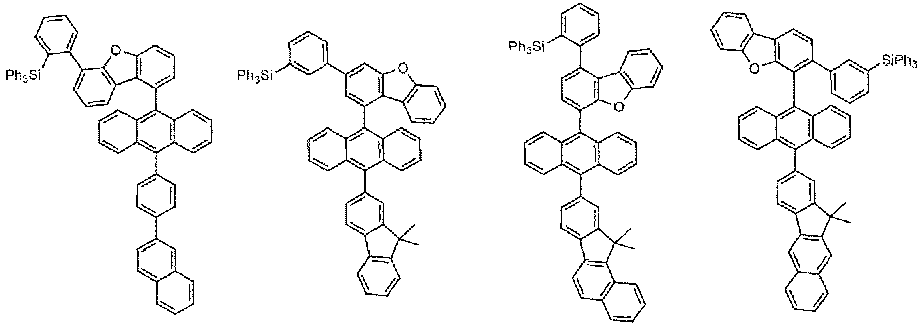

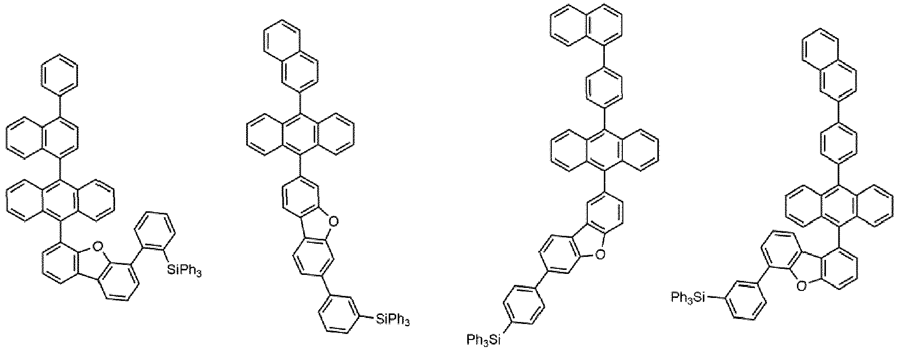

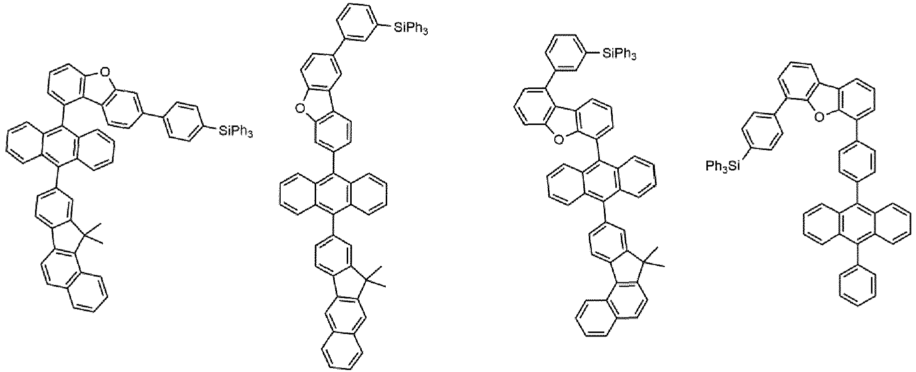

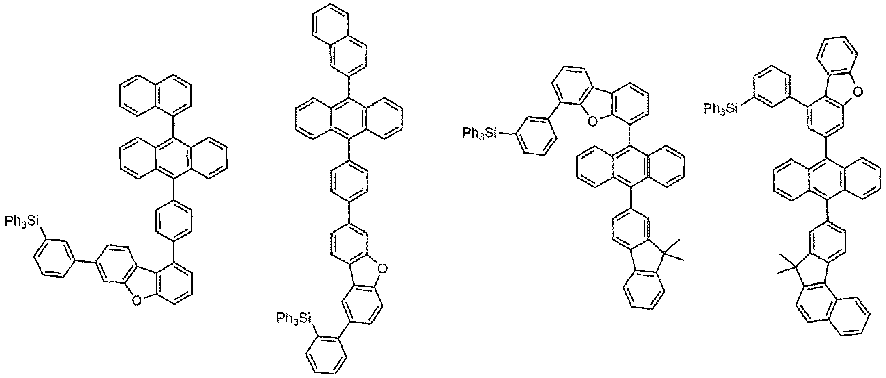

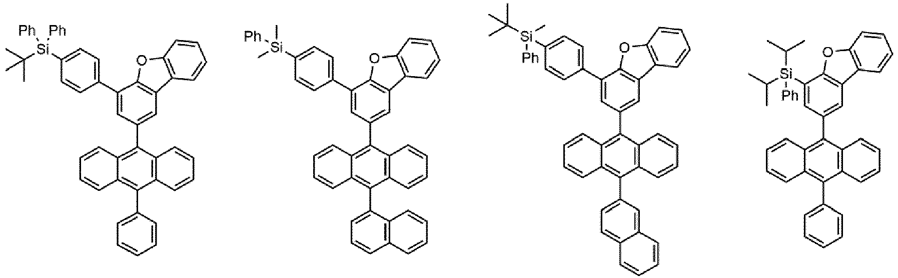

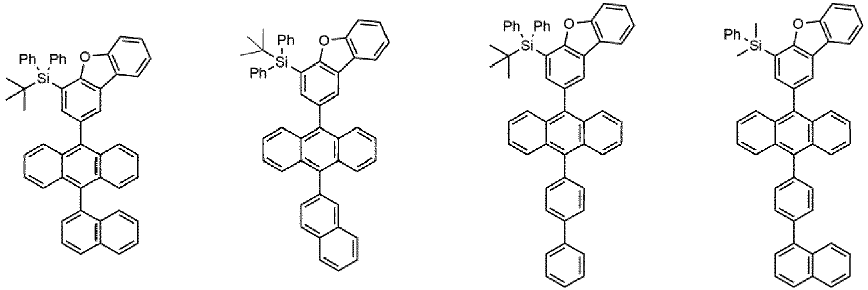

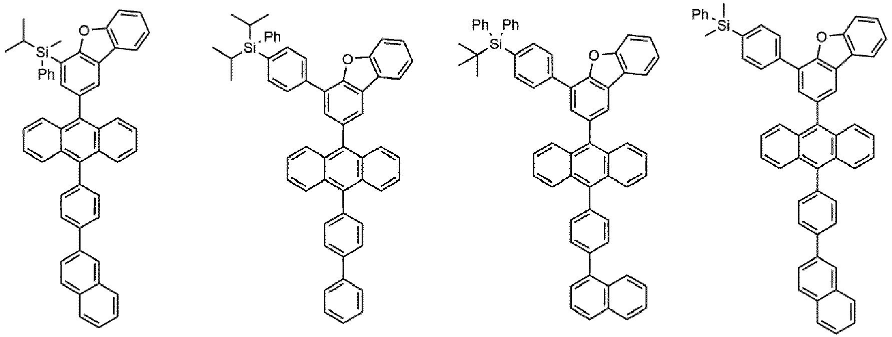

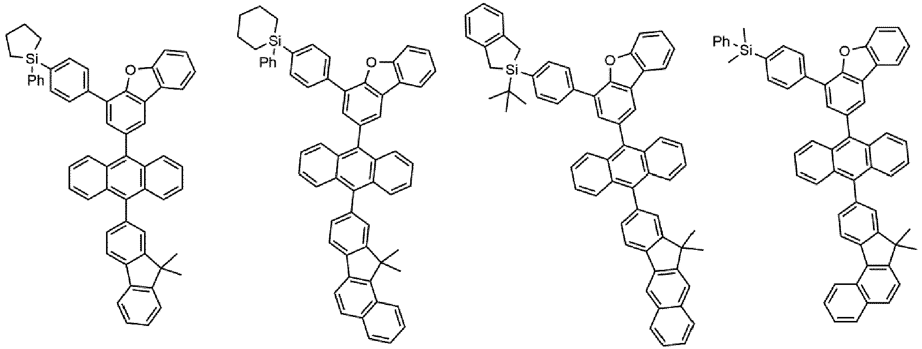

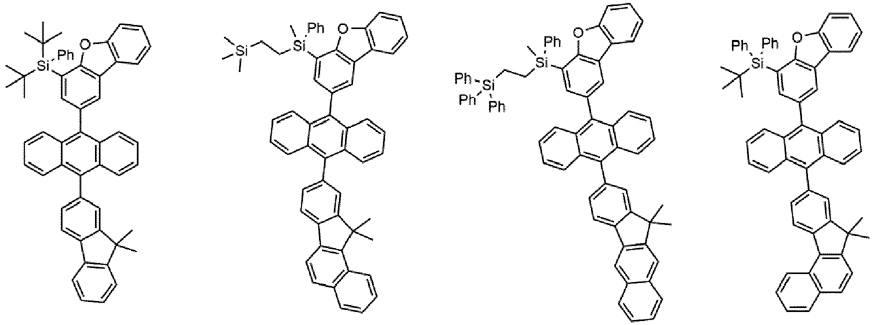

Meanwhile, the compound represented by Chemical Formula 1 can be prepared, for example, according to the preparation method as shown in the following Reaction Scheme 1. In Reaction Scheme 1 below, a to c, R₁ to R₆, L₁, L₂, and Ar are as defined above.

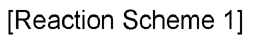

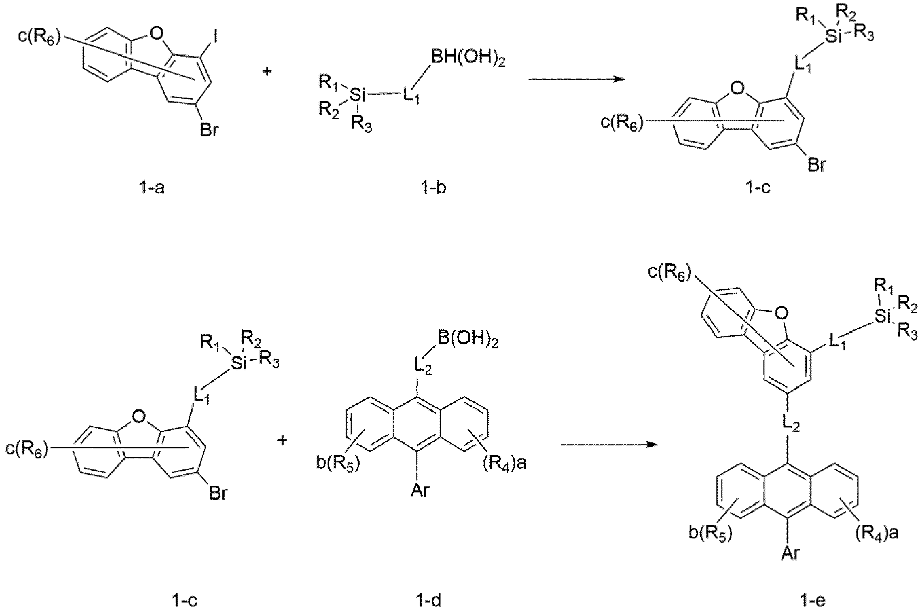

Specifically, in Reaction Scheme 1, Compound 1-c is synthesized through a coupling reaction between the compound represented by Chemical Formula 1-a and the compound represented by Chemical Formula 1-b, and a compound represented by Chemical Formula 1-e is prepared through a coupling reaction between the compound represented by Chemical Formula 1-c and the compound represented by Chemical Formula 1-d.

The aforementioned reaction is a Suzuki coupling reaction, which is preferably carried out in the presence of a palladium catalyst and a base, and a reactive group for the Suzuki coupling reaction can be changed as known in the art.

The above preparation method may be further specified in the synthesis examples described hereinafter.

In another embodiment of the invention, there is provided a coating composition including the compound represented by Chemical Formula 1.

In the other embodiment of the invention, there is provided an organic light emitting device including a compound represented by Chemical Formula 1 described above.

As an example, there is provided an organic light emitting device including a first electrode; a second electrode provided opposite to the first electrode; and one or more organic material layers provided between the first electrode and the second electrode, wherein one or more layers of the organic material layers include the compound represented by Chemical Formula 1.

The compound represented by Chemical Formula 1 introduces a bulky structure of a silane group to increase the molecular asymmetry and thereby exhibit improved solubility as compared with the structure of a conventional anthracene derivative.

Therefore, the solution processability is increased, and thus efficiency and lifetime can be improved when manufacturing an organic light emitting device including the compound represented by Chemical Formula 1.

In addition, the glass transition temperature of the material at the time of introduction of the silane group increases, and thus the stability of the film at the time of manufacturing the film also increases.

Therefore, when manufacturing an organic light emitting device including the compound represented by Chemical Formula 1, it is possible to expect high efficiency and a long lifetime.

The organic material layer of the organic light emitting device of the present invention may have a single-layer structure, or it may have a multilayered structure in which two or more organic material layers are stacked.

For example, the organic light emitting device of the present invention may have a structure including a hole injection layer, a hole transport layer, a light emitting layer, an electron transport layer, an electron injection layer, and the like as the organic material layer.

However, the structure of the organic light emitting device is not limited thereto, and it may include a smaller number of organic layers.

Further, the organic material layer may include a hole injection layer, a hole transport layer, or a layer for simultaneously performing hole injection and transport, wherein the hole injection layer, the hole transport layer, or the layer for simultaneously performing hole injection and transport may include the compound represented by Chemical Formula 1.

The organic material layer may include a light emitting layer, wherein the light emitting layer may include the compound represented by Chemical Formula 1.

In this case, the compound represented by Chemical Formula 1 can be used as a host material in the light emitting layer. More specifically, the compound represented by Chemical Formula 1 may be used as a host used in the light emitting layer of the blue organic light emitting device.

The organic material layer may include an electron transport layer or an electron injection layer, wherein the electron transport layer or the electron injection layer may include the compound represented by Chemical Formula 1.

The electron transport layer, the electron injection layer, or the layer for simultaneously performing electron transport and electron injection may include the compound represented by Chemical Formula 1.

The organic material layer may include a light emitting layer and an electron transport layer, wherein the electron transport layer may include the compound represented by Chemical Formula 1.

The organic light emitting device according to the present invention may be a normal type of organic light emitting device in which an anode, one or more organic material layers, and a cathode are sequentially stacked on a substrate.

The organic light emitting device according to the present invention may be an inverted type of organic light emitting device in which a cathode, one or more organic material layers, and an anode are sequentially stacked on a substrate.

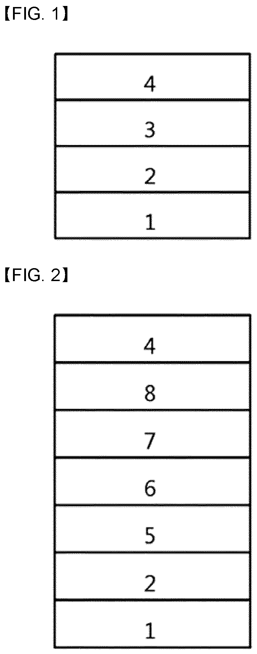

For example, the structure of an organic light emitting device according to an embodiment of the present invention is illustrated in FIGS. 1 and 2.

FIG. 1 shows an example of an organic light emitting device including a substrate 1, an anode 2, a light emitting layer 3, and a cathode 4.

In such a structure, the compound represented by Chemical Formula 1 may be included in the light emitting layer.

FIG. 2 shows an example of an organic light emitting device including a substrate 1, an anode 2, a hole injection layer 5, a hole transport layer 6, a light emitting layer 7, an electron transport layer 8, and a cathode 4.

In such a structure, the compound represented by Chemical Formula 1 may be included in one or more layers of the hole injection layer, the hole transport layer, the light emitting layer and the electron transport layer.

The organic light emitting device according to the present invention may be manufactured by materials and methods known in the art, except that one or more layers of the organic material layers include the compound represented by Chemical Formula 1.

Moreover, when the organic light emitting device includes a plurality of organic material layers, the organic material layers may be formed of the same material or different materials.

For example, the organic light emitting device according to the present invention can be manufactured by sequentially stacking a first electrode, an organic material layer, and a second electrode on a substrate.

In this case, the organic light emitting device may be manufactured by depositing a metal, metal oxides having conductivity, or an alloy thereof on the substrate to form an anode, forming organic material layers including the hole injection layer, the hole transport layer, the light emitting layer, and the electron transport layer thereon, and then depositing a material that can be used as the cathode thereon, using a PVD (physical vapor deposition) method such as a sputtering method or an e-beam evaporation method.

In addition to such a method, the organic light emitting device may be manufactured by sequentially depositing a cathode material, an organic material layer, and an anode material on a substrate.

Further, the compound represented by Chemical Formula 1 may be formed into an organic material layer by a solution coating method as well as a vacuum deposition method at the time of manufacturing an organic light emitting device.

In particular, the compound represented by Chemical Formula 1 has excellent solubility in the solvent used in a solution coating method and thus is easy to apply to the solution coating method. Herein, the solution coating method means spin coating, dip coating, doctor blading, inkjet printing, screen printing, a spray method, roll coating, or the like, but is not limited thereto.

Thus, one embodiment of the present invention provides a coating composition including the compound represented by Chemical Formula 1 and a solvent.

The solvent is not particularly limited as long as it is capable of dissolving or dispersing the compound according to one embodiment of the present invention. Examples of the solvent may include chlorine-based solvents such as chloroform, methylene chloride, 1,2-dichloroethane, 1,1,2-trichloroethane, chlorobenzene, and o-dichlorobenzene; ether-based solvents such as tetrahydrofuran and dioxane; aromatic hydrocarbon-based solvents such as toluene, xylene, trimethylbenzene, and mesitylene; aliphatic hydrocarbon-based solvents such as cyclohexane, methylcyclohexane, n-pentane, n-hexane, n-heptane, n-octane, n-nonane, and n-decane; ketone-based solvents such as acetone, methyl ethyl ketone, and cyclohexanone; ester-based solvents such as ethyl acetate, butyl acetate, and ethyl cellosolve acetate; polyhydric alcohols such as ethylene glycol, ethylene glycol monobutyl ether, ethylene glycol monoethyl ether, ethylene glycol monomethyl ether, dimethoxyethane, propylene glycol, diethoxymethane, triethylene glycol monoethyl ether, glycerin, and 1,2-hexanediol, and derivatives thereof; alcohol-based solvents such as methanol, ethanol, propanol, isopropanol, and cyclohexanol; sulfoxide-based solvents such as dimethyl sulfoxide; amide-based solvents such as N-methyl-2-pyrrolidone and N,N-dimethylformamide; benzoate-based solvents such as butyl benzoate and methyl-2-methoxybenzoate; tetralin; 3-phenoxy-toluene; and the like.

Further, the above-mentioned solvents can be used alone or in combination of two or more thereof.

In addition, the viscosity of the coating composition is preferably 1 cP to 10 cP, and coating is easy within the above range.

Further, in the coating composition, the concentration of the compound according to the present invention is preferably 0.1 wt/v% to 20 wt/v%.

Another embodiment of the present invention provides a method of forming a functional layer using the coating composition described above.

Specifically, the method includes a step of coating the coating composition according to the present invention described above by a solution process, and a step of heat-treating the coated coating composition.

In the heat treatment step, the heat treatment temperature is preferably 150 to 230 °C.

Further, the heat treatment time is preferably 1 minute to 3 hours, more preferably 10 minutes to 1 hour.

The heat treatment is preferably performed in an inert gas atmosphere such as argon or nitrogen.

In addition to such a method, the organic light emitting device may be manufactured by sequentially depositing a cathode material, an organic material layer, and an anode material on a substrate (International Publication WO2003/012890).

However, the manufacturing method is not limited thereto.

As an example, the first electrode is an anode and the second electrode is a cathode, or alternatively, the first electrode is a cathode and the second electrode is an anode.

As the anode material, generally, a material having a large work function is preferably used so that holes can be smoothly injected into the organic material layer.

Specific examples of the anode material include metals such as vanadium, chromium, copper, zinc, and gold, or an alloy thereof; metal oxides such as zinc oxides, indium oxides, indium tin oxides (ITO), and indium zinc oxides (IZO); a combination of metals and oxides, such as ZnO:AI or SnO₂:Sb; conductive polymers such as poly(3-methylthiophene), poly[3,4-(ethylene-1,2-dioxy)thiophene] (PEDOT), polypyrrole, and polyaniline; and the like, but are not limited thereto.

As the cathode material, generally, a material having a small work function is preferably used so that electrons can be easily injected into the organic material layer.

Specific examples of the cathode material include metals such as magnesium, calcium, sodium, potassium, titanium, indium, yttrium, lithium, gadolinium, aluminum, silver, tin, and lead, or alloys thereof; and a multilayered structure material such as LiF/AI or LiO₂/Al, and the like, but are not limited thereto.

The hole injection layer is a layer for injecting holes from the electrode, and the hole injection material is preferably a compound which has a capability of transporting the holes, thus has a hole injecting effect in the anode and an excellent hole-injecting effect to the light emitting layer or the light emitting material, prevents excitons produced in the light emitting layer from moving to an electron injection layer or the electron injection material, and is excellent in the ability to form a thin film.

It is preferable that a HOMO (highest occupied molecular orbital) of the hole injection material is between the work function of the anode material and a HOMO of a peripheral organic material layer.

Specific examples of the hole injection material include metal porphyrin, oligothiophene, an arylamine-based organic material, a hexanitrilehexaazatriphenylene-based organic material, a quinacridone-based organic material, a perylene-based organic material, an anthraquinone, polyaniline, and polythiophene-based conductive polymer, and the like, but are not limited thereto.

The hole transport layer is a layer that receives holes from a hole injection layer and transports the holes to the light emitting layer. The hole transport material is suitably a material having large mobility to the holes, which may receive holes from the anode or the hole injection layer and transfer the holes to the light emitting layer.

Specific examples thereof include an arylamine-based organic material, a conductive polymer, a block copolymer in which a conjugate portion and a non-conjugate portion are present together, and the like, but are not limited thereto.

The light emitting material is preferably a material which may receive holes and electrons transported from a hole transport layer and an electron transport layer, respectively, and combine the holes and the electrons to emit light in a visible ray region, and has good quantum efficiency to fluorescence or phosphorescence.

Specific examples thereof include an 8-hydroxy-quinoline aluminum complex (Alq₃); a carbazole-based compound; a dimerized styryl compound; BAlq; a 10-hydroxybenzoquinoline-metal compound; a benzoxazole, benzothiazole, and benzimidazole-based compound; a poly(p-phenylene vinylene)(PPV)-based polymer; a spiro compound; polyfluorene, rubrene, and the like, but are not limited thereto.

The light emitting layer may include a host material and a dopant material. The host material may include a fused aromatic ring derivative, a heterocyclic-containing compound, or the like. Specifically, the fused aromatic ring derivatives may be anthracene derivatives, pyrene derivatives, naphthalene derivatives, pentacene derivatives, phenanthrene compounds, fluoranthene compounds, and the like, and the heterocyclic-containing compounds may be carbazole derivatives, dibenzofuran derivatives, ladder-type furan compounds, pyrimidine derivatives, and the like, but are not limited thereto. Preferably, the compound according to the present invention is used as the host material.

The dopant material may be an aromatic amine derivative, a styrylamine compound, a boron complex, a fluoranthene compound, a metal complex, and the like.

Specifically, the aromatic amine derivative is a substituted or unsubstituted fused aromatic ring derivative having an arylamino group, and examples thereof include pyrene, anthracene, chrysene, periflanthene, and the like, which have an arylamino group. The styrylamine compound is a compound where at least one arylvinyl group is substituted in a substituted or unsubstituted arylamine, in which one or more substituent groups selected from the group consisting of an aryl group, a silyl group, an alkyl group, a cycloalkyl group, and an arylamino group are substituted or unsubstituted. Specific examples thereof include styrylamine, styryl diamine, styryl triamine, styryl tetramine, and the like, but are not limited thereto.

Further, the metal complex includes an iridium complex, a platinum complex, and the like, but is not limited thereto.

The electron transport layer is a layer which receives electrons from an electron injection layer and transports the electrons to a light emitting layer, and an electron transport material is suitably a material which can receive electrons well from a cathode and transfer the electrons to a light emitting layer and has large mobility for electrons.

Specific examples thereof include: an Al complex of 8-hydroxyquinoline; a complex including Alq₃; an organic radical compound; a hydroxyflavone-metal complex; and the like, but are not limited thereto.

The electron transport layer may be used with any desired cathode material, as used according to the related art.

In particular, appropriate examples of the cathode material are a typical material which has a low work function, followed by an aluminum layer or a silver layer.

Specific examples thereof include cesium, barium, calcium, ytterbium, and samarium, in each case followed by an aluminum layer or a silver layer.

The electron injection layer is a layer which injects electrons from an electrode, and is preferably a compound which has a capability of transporting electrons, has an effect of injecting electrons from a cathode and an excellent effect of injecting electrons into a light emitting layer or a light emitting material, prevents excitons produced from the light emitting layer from moving to a hole injection layer, and is also excellent in the ability to form a thin film.

Specific examples thereof include fluorenone, anthraquinodimethane, diphenoquinone, thiopyran dioxide, oxazole, oxadiazole, triazole, imidazole, perylenetetracarboxylic acid, fluorenylidene methane, anthrone, and the like, and derivatives thereof, a metal complex compound, a nitrogen-containing 5-membered ring derivative, and the like, but are not limited thereto.

Examples of the metal complex compound include 8-hydroxyquinolinato lithium, bis(8-hydroxyquinolinato)zinc, bis(8-hydroxyquinolinato)copper, bis(8-hydroxyquinolinato)manganese, tris(8-hydroxyquinolinato)aluminum, tris(2-methyl-8-hydroxyquinolinato)aluminum, tris(8-hydroxyquinolinato)gallium, bis(10-hydroxybenzo[h]quinolinato)beryllium, bis(10-hydroxybenzo[h]quinolinato)zinc, bis(2-methyl-8-quinolinato)chlorogallium, bis(2-methyl-8-quinolinato)(o-cresolato)gallium, bis(2-methyl-8-quinolinato)(1-naphtholato)aluminum, bis(2-methyl-8-quinolinato)(2-naphtholato)gallium, and the like, but are not limited thereto.

The organic light emitting device according to the present invention may be a front side emission type, a back side emission type, or a double-sided emission type according to the used material.

In addition, the compound represented by Chemical Formula 1 may be included in an organic solar cell or an organic transistor in addition to an organic light emitting device.

The preparation of the compound represented by Chemical Formula 1 and the organic light emitting device containing the same will be described in detail in the following examples.

However, these examples are presented for illustrative purposes only and are not intended to limit the scope of the present invention.

### Example 1: Synthesis of Compound A

### Step 1-1) Synthesis of Compound 3

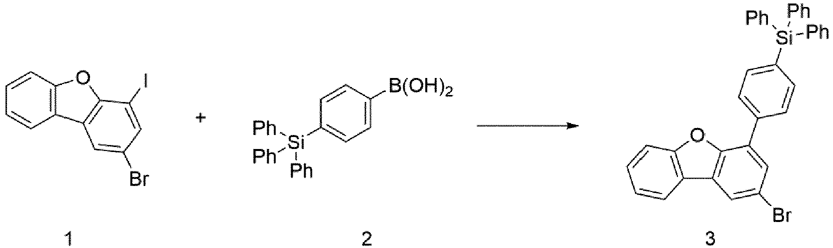

Compound 1 (11.2 g, 30 mmol), Compound 2 (11.4 g, 30 mmol), Pd(PPh₃)₄ (1.73 g, 1.5 mmol), and K₂CO₃ (8.28 g, 60 mmol) were dissolved in toluene (240 ml) and distilled water (60 ml) and then stirred at 90 °C for 15 hours.

The organic layer was separated, and then water was removed using MgSO₄ and subjected to reduced pressure to remove the solvent.

The resulting material was subjected to column chromatography using dichloromethane and hexane to separate and purify Compound 3 (11.1 g, 64 %).

MS: [M+H]⁺ = 581

### Step 1-2) Synthesis of Compound A

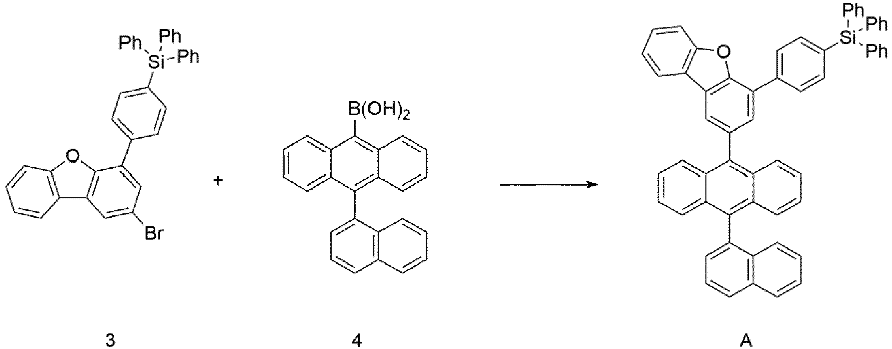

Compound 3 (2 g, 3.44 mmol), Compound 4 (1.8 g, 5.16 mmol), Pd(PPh₃)₄ (196 mg, 0.17 mmol), and K₂CO₃ (1.43 g, 10.3 mmol) were dissolved in toluene (30 ml) and distilled water (10 ml) and then stirred at 90 °C for 15 hours. The organic layer was separated, and then water was removed using MgSO₄ and subjected to reduced pressure to remove the solvent.

The resulting material was subjected to column chromatography using dichloromethane and hexane to separate and purify Compound A (1.1 g, 40 %).

MS: [M+H]⁺ = 805

### Example 2: Synthesis of Compound B

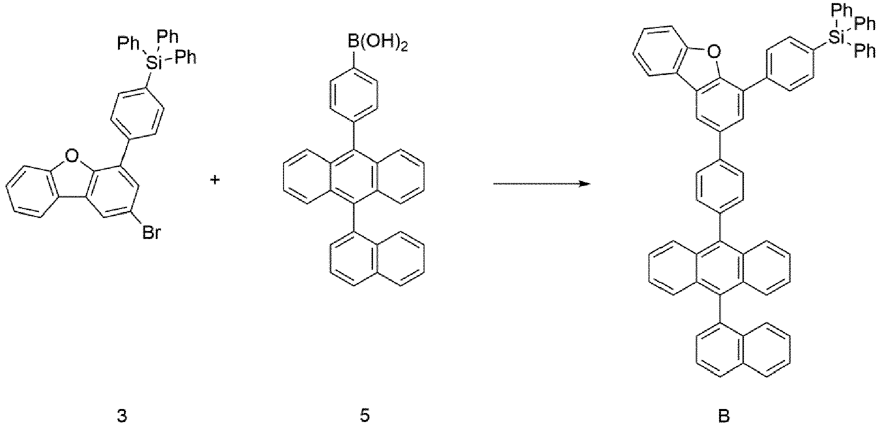

Compound B was prepared in the same manner as in Step 1-2 of Example 1 except that Compound 5 (2.2 g, 5.16 mmol) was used instead of Compound 4.

MS: [M+H]⁺ = 881

### Example 3: Synthesis of Compound C

### Step 3-1) Synthesis of Compound 6

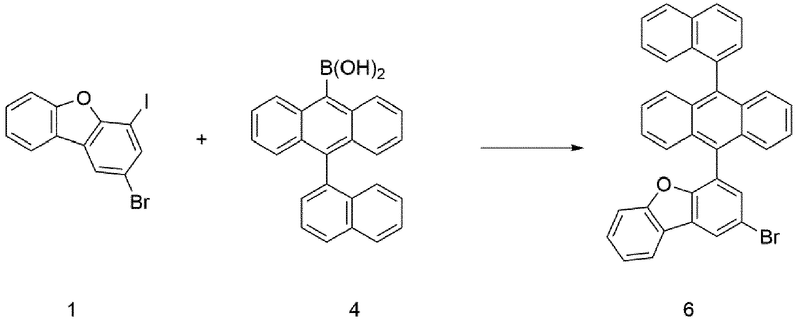

Compound 6 was prepared in the same manner as in Step 1-1 of Example 1 except that Compound 4 (10.4 g, 30 mmol) was used instead of Compound 2.MS: [M+H]⁺ = 549

### Step 3-2) Synthesis of Compound C

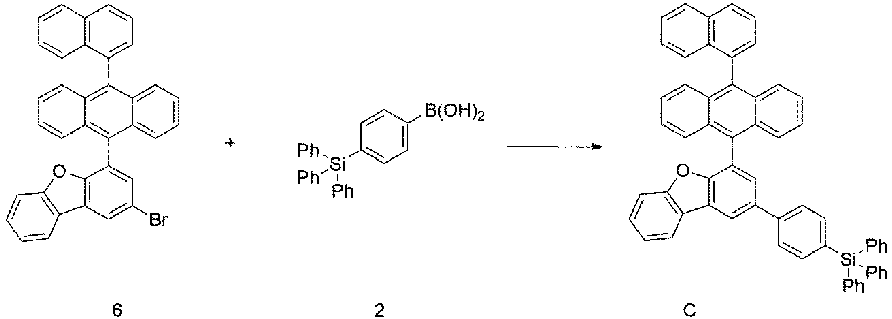

Compound 6 (2.7 g 5 mmol), Compound 2 (2.9 g, 7.5 mmol), Pd(PPh₃)₄ (289 mg, 0.25 mmol), and K₂CO₃ (2.1 g, 15 mmol) were dissolved in toluene (45 ml) and distilled water (15 ml) and then stirred at 90 °C for 15 hours.

The organic layer was separated, and then water was removed using MgSO₄ and subjected to reduced pressure to remove the solvent.

The resulting material was subjected to column chromatography using dichloromethane and hexane to separate and purify Compound C.

MS: [M+H]⁺ = 805

### [Experimental Example 1]

A glass substrate on which ITO (indium tin oxide) was coated as a thin film to a thickness of 50 nm was put into distilled water in which a detergent was dissolved, and ultrasonically cleaned.

In this case, a product manufactured by Fischer Co. was used as the detergent, and as the distilled water, distilled water filtered twice using a filter manufactured by Millipore Co. was used.

After the ITO was cleaned for 30 minutes, ultrasonic cleaning was repeated twice using distilled water for 10 minutes each. After the cleaning with distilled water was completed, the substrate was ultrasonically cleaned with solvents of isopropyl alcohol, acetone, and methanol, then dried and transferred to a plasma cleaner. In addition, the substrate was cleaned for 5 minutes using oxygen plasma, and then transferred to a vacuum depositor.

On the ITO transparent electrode thus prepared, an aqueous dispersion of a conductive polymer and a sulfonic acid polymer was spin-coated at 1000 rpm for 60 seconds, baked at 80 °C for 2 minutes, and baked at 120 °C for 15 minutes to form a hole injection layer.

A solution of a triarylamine polymer was spin-coated on the hole injection layer and baked to form a hole transport layer.

A 2 wt% toluene solution was prepared by using the compound A prepared in Example 1 and the following Compound BD1 at a weight ratio of 95:5, spin-coated on the hole transport layer at 5000 rpm, baked at 80 °C for 2 minutes, and baked at 120 °C for 30 minutes to form a light emitting layer.

This was dried at 130 °C for 10 minutes under a nitrogen gas atmosphere, and then lithium fluoride (LiF) was deposited to a film thickness of about 1 nm, and finally, aluminum was deposited to a film thickness of 100 nm to form a cathode.

In the above-mentioned process, the deposition rate of lithium fluoride of the cathode was maintained at 0.3 Å/s, the deposition rate of aluminum was maintained at 2 Å/s, and the degree of vacuum during the deposition was maintained at 2.67×10⁻⁵ to 6.67×10⁻⁴ Pa (2×10⁻⁷ to 5×10⁻⁶ Torr), thereby manufacturing an organic light emitting device.

Device structure: ITO (50 nm)/HIL (40 nm)/HTL (20 nm)/EML (55 nm)/LiF (1 nm)/AI (100 nm)

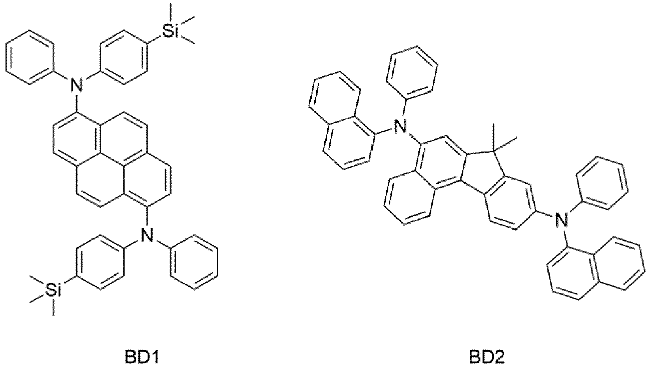

### [Experimental Examples 2 to 6]

Organic light emitting devices were manufactured in the same manner as in Experimental Example 1, except that the compounds used in the preparation of the light emitting layer were changed as shown in Table 1 below.

### [Comparative Experimental Examples 1 to 41

The organic light emitting devices were manufactured in the same manner as in Experimental Example 1, except that the compounds used in the preparation of the light emitting layer were changed as shown in Table 1 below.

In Table 1, Compound X1 and Compound X2 are as follows.

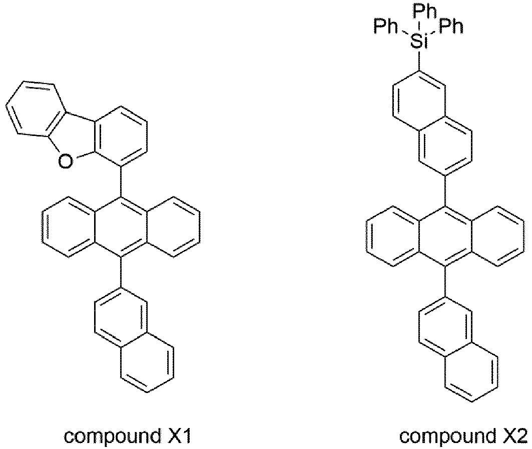

The organic light emitting devices manufactured in Experimental Examples 1 to 6 and Comparative Experimental Examples 1 to 4 were measured for the driving voltage, current efficiency, and quantum efficiency values at a current density of 10 mA/cm², the time required for the luminance to be reduced to 95 % of the initial luminance at a current density of 10 mA/cm² was measured, and the results are shown in Table 1 below.

**[Table 1]**

| | Light emitting layer compound | | Voltage (V) | Current efficiency (Im/W) | Emission efficiency (cd/A) | Quantum efficiency (%) | Lifetime (h) |
|---|---|---|---|---|---|---|---|
| Experimental Example 1 | Compound A | Compound BD1 | 4.53 | 5.12 | 7.39 | 7.61 | 18 |
| Experimental Example 2 | Compound A | Compound BD2 | 4.46 | 5.42 | 7.69 | 7.89 | 27 |
| Experimental Example 3 | Compound B | Compound BD1 | 4.53 | 5.20 | 7.50 | 7.69 | 17 |
| Experimental Example 4 | Compound B | Compound BD2 | 4.48 | 5.18 | 7.37 | 7.16 | 21 |
| Experimental Example 5 | Compound C | Compound BD1 | 4.52 | 5.35 | 7.70 | 7.92 | 24 |
| Experimental Example 6 | Compound C | Compound BD2 | 4.42 | 5.66 | 7.97 | 7.68 | 22 |
| Comparative Experimental Example 1 | Compound X1 | Compound BD1 | 4.48 | 5.69 | 8.10 | 7.87 | 5 |
| Comparative Experimental Example 2 | Compound X1 | Compound BD2 | 4.43 | 5.58 | 7.87 | 7.77 | 10 |
| Comparative Experimental Example 3 | Compound X2 | Compound BD1 | 4.62 | 5.34 | 7.42 | 7.69 | 14 |
| Comparative Experimental Example 4 | Compound X2 | Compound BD2 | 4.68 | 5.54 | 7.59 | 7.45 | 17 |

As shown in Table 1 above, it was confirmed that the organic light emitting device using the compound of the present invention as a host of the light-emitting layer exhibited excellent characteristics in terms of voltage, efficiency, and lifetime.

### [DESCRIPTION OF SYMBOLS]

1: substrate 2: anode
3: light emitting layer 4: cathode
5: hole injection layer 6: hole transport layer
7: light emitting layer 8: electron transport layer

## Claims

1. A compound represented by the following Chemical Formula 1: 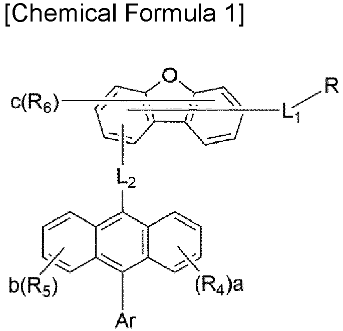 wherein, in Chemical Formula 1,
R is Si(R₁)(R₂)(R₃),
R₁ to R₃ are each independently hydrogen; a substituted or unsubstituted C₁₋₆₀ alkyl; a substituted or unsubstituted C₆₋₆₀ aryl; a (tri(C₁₋₆₀ alkyl)silyl)-(C₁₋₁₀ alkylene)-; or a (tri(C₆₋₆₀ aryl)silyl)-(C₁₋₁₀ alkylene)-, or R₁ and R₂ are linked together to form a ring, provided that the case wherein R₁ to R₃ are all a substituted or unsubstituted C₁₋₆₀ alkyl is excluded,
a to c are each independently an integer of 0 to 4,
R₄ to R₆ are each independently hydrogen; deuterium; a halogen; a cyano; a substituted or unsubstituted C₁₋₆₀ alkyl; a substituted or unsubstituted C₁₋₆₀ alkoxy; a substituted or unsubstituted C₁₋₆₀ thioalkyl; a substituted or unsubstituted C₃₋₆₀ cycloalkyl; a substituted or unsubstituted C₆₋₆₀ aryl; or a substituted or unsubstituted C₅₋₆₀ heteroaryl containing one or more heteroatoms selected from the group consisting of N, O, and S,
L₁ is a single bond; or phenylene,
L₂ is a single bond; or a substituted or unsubstituted C₆₋₆₀ arylene, and
Ar is a substituted or unsubstituted C₆₋₆₀ aryl; or a substituted or unsubstituted C₅₋₆₀ heteroaryl containing one or more heteroatoms selected from the group consisting of N, O, and S,
wherein the term "substituted" means substituted with one or more substituents selected from the group consisting of deuterium; a halogen group; a nitrile group; a nitro group; a hydroxy group; a carbonyl group; an ester group; an imide group; an amino group; a phosphine oxide group; an alkoxy group; an aryloxy group; an alkylthioxy group; an arylthioxy group; an alkylsulfoxy group; an arylsulfoxy group; a silyl group; a boron group; an alkyl group; a cycloalkyl group; an alkenyl group; an aryl group; an aralkyl group; an aralkenyl group; an alkylaryl group; an alkylamine group; an aralkylamine group; a heteroarylamine group; an arylamine group; an arylphosphine group; and a heterocyclic group containing at least one of N, O, and S atoms, or being substituted with a substituent to which two or more substituents are linked among said substituents.

2. The compound according to claim 1, wherein
Chemical Formula 1 is represented by the following Chemical Formula 1-1 or 1-2: 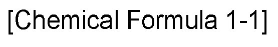 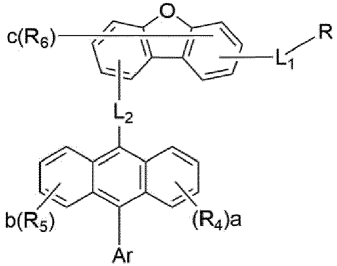 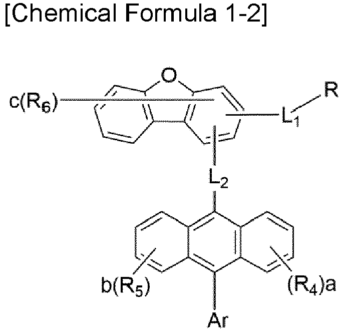 wherein, in Chemical Formula 1-1 or 1-2,
R, a to c, R₄ to R₆, L₁, L₂, and Ar are as defined in claim 1.

3. The compound according to claim 1, wherein
a to c are all 0.

4. The compound according to claim 1, wherein
R₁ to R₃ are each independently methyl; i-propyl; t-butyl; phenyl; trimethylsilyl-ethylene; or triphenylsilyl-ethylene, or R₁ and R₂ are linked together to form a cyclopentyl, cyclohexyl, or 2,3-dihydro-1H-indenyl ring.

5. The compound according to claim 1, wherein
at least one of R₁ to R₃ is phenyl.

6. The compound according to claim 1, wherein
L₂ is a single bond or phenylene.

7. The compound according to claim 1, wherein
Ar is any one selected from the group consisting of the following:

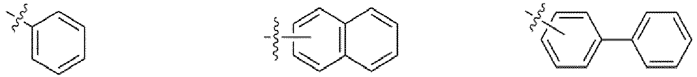

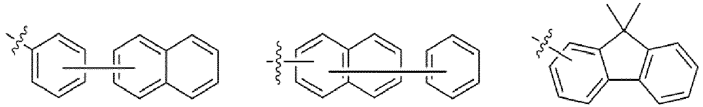

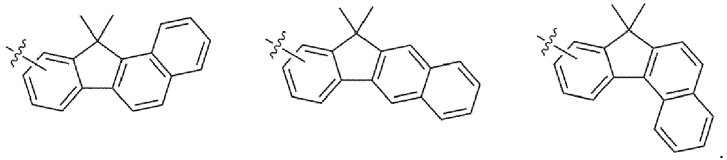

8. The compound according to claim 1, wherein
the compound represented by the Chemical Formula 1 is selected from the group consisting of the following:

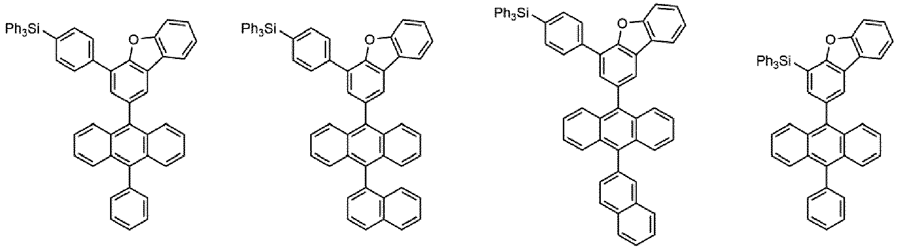

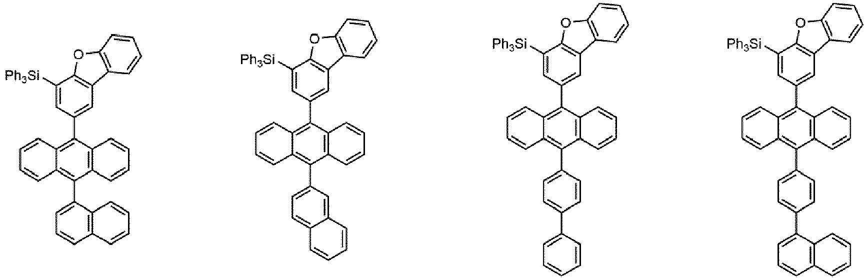

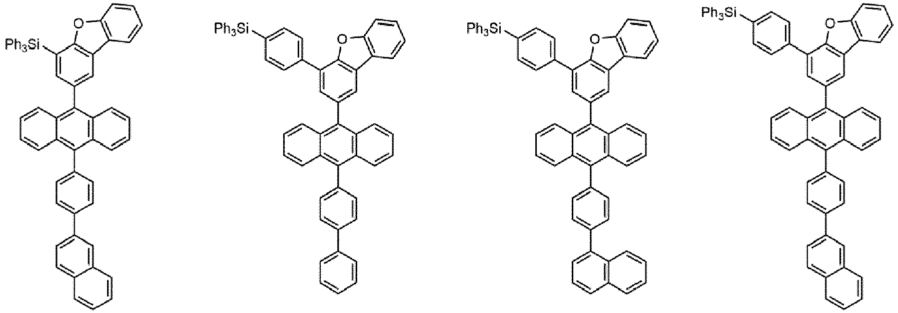

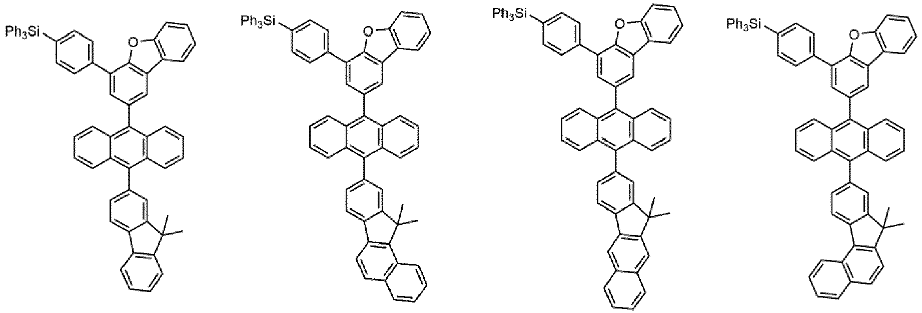

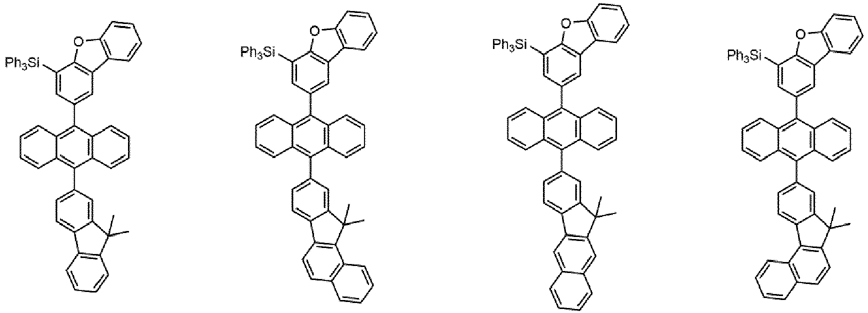

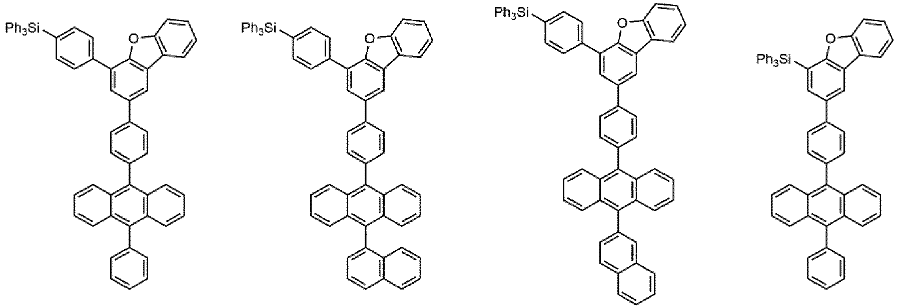

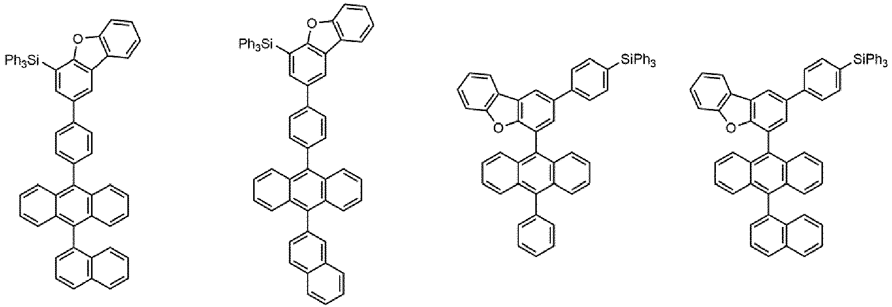

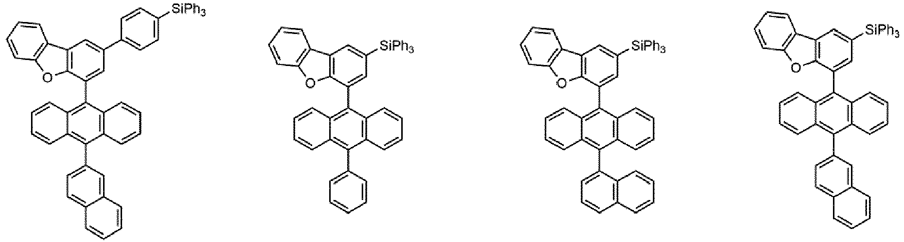

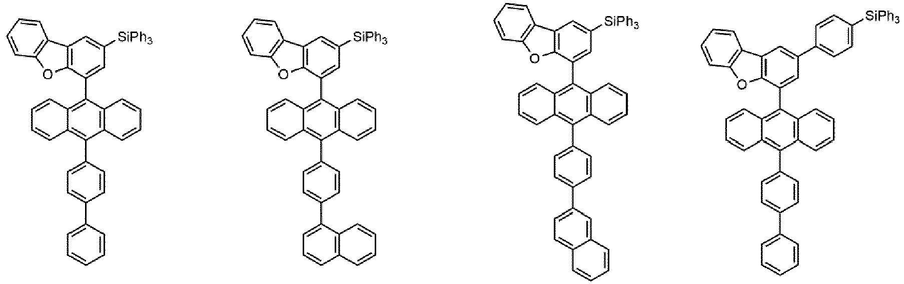

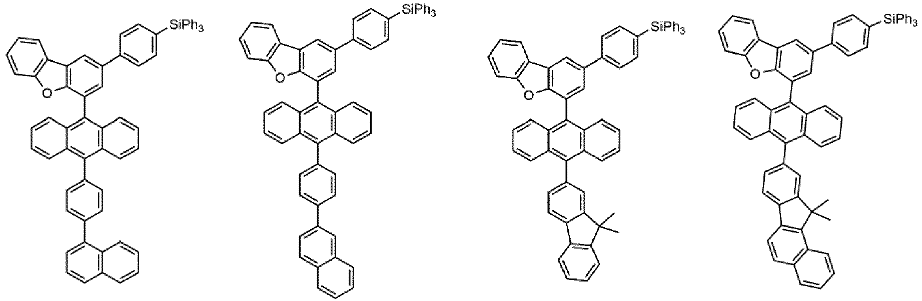

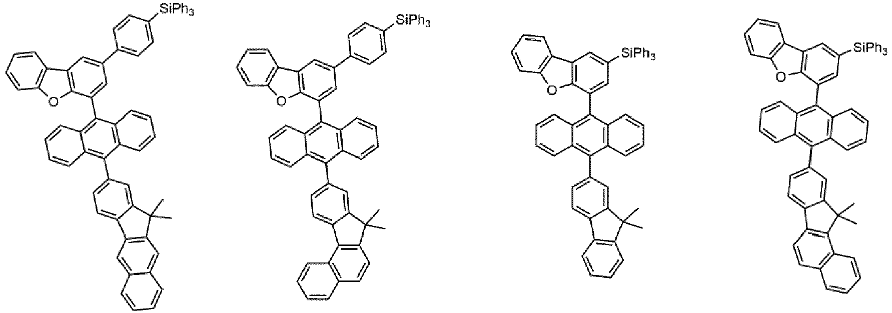

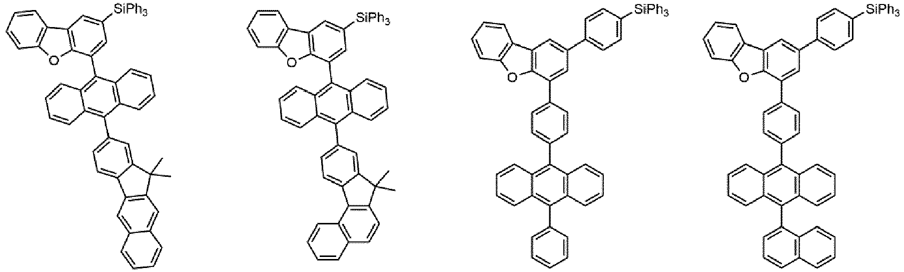

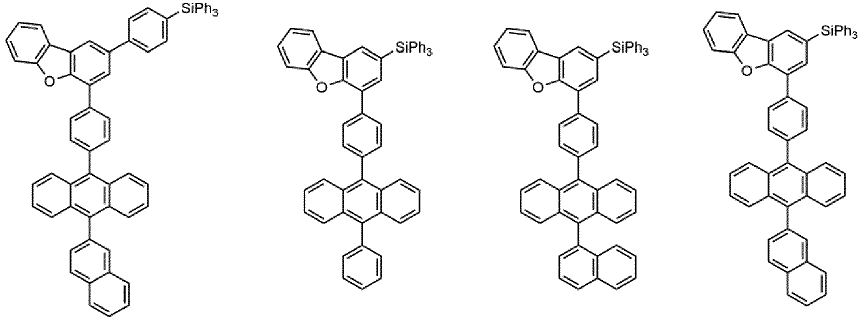

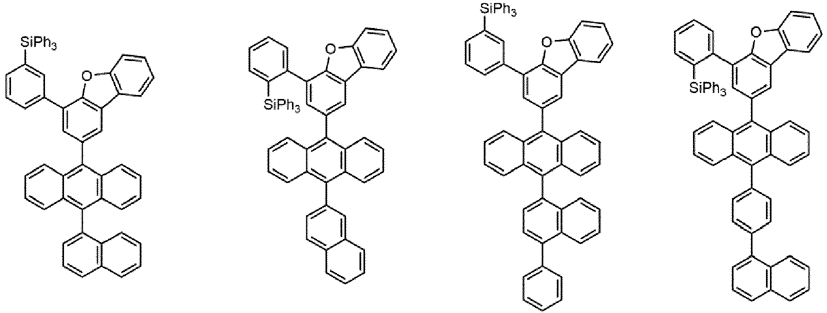

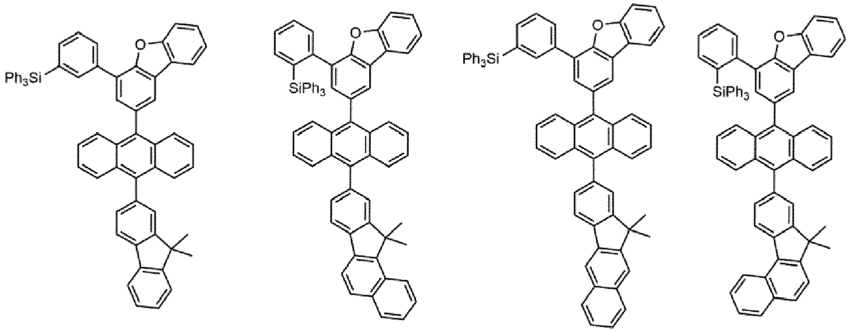

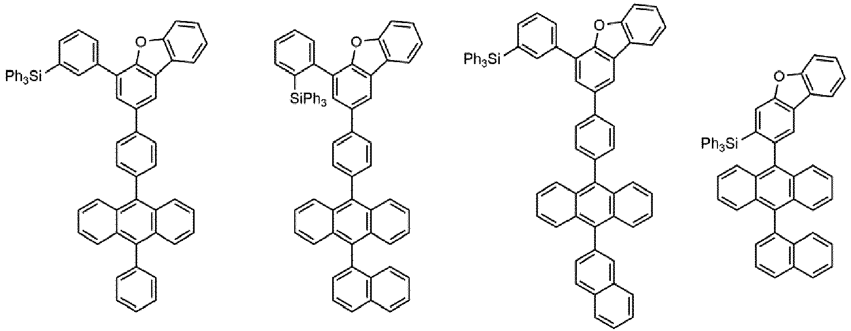

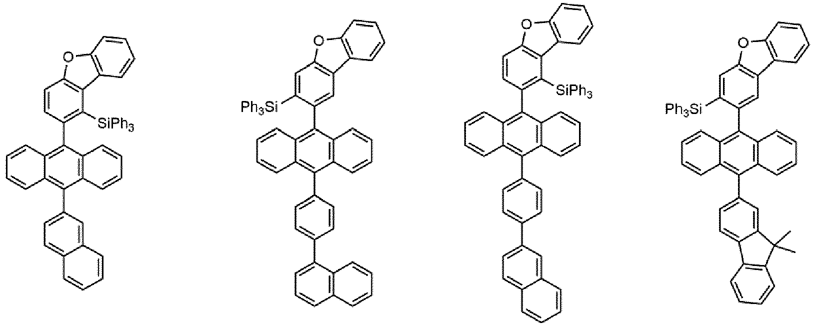

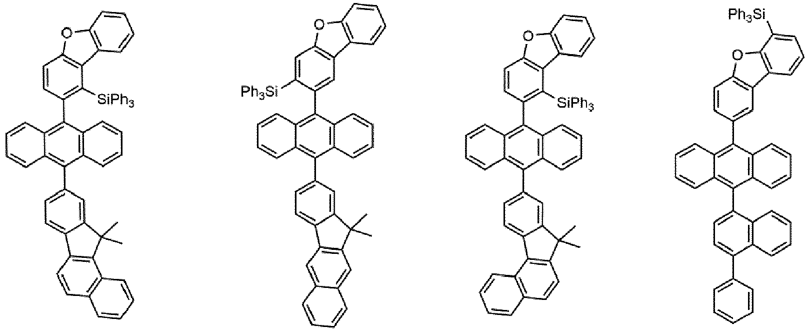

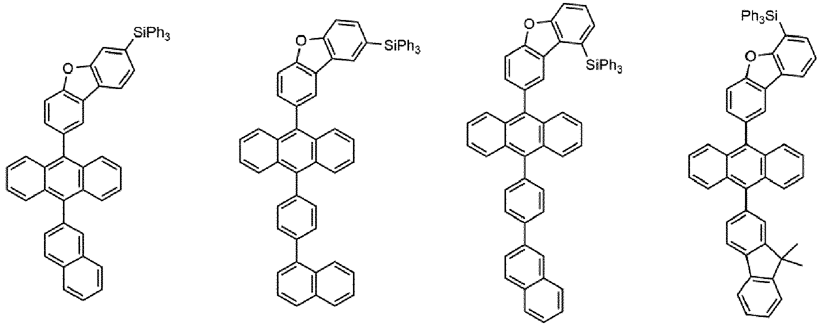

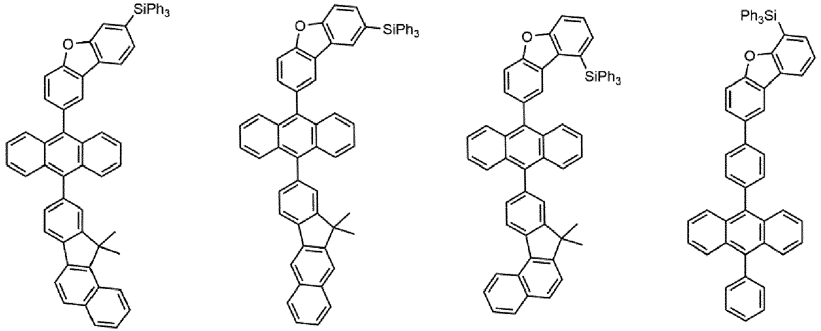

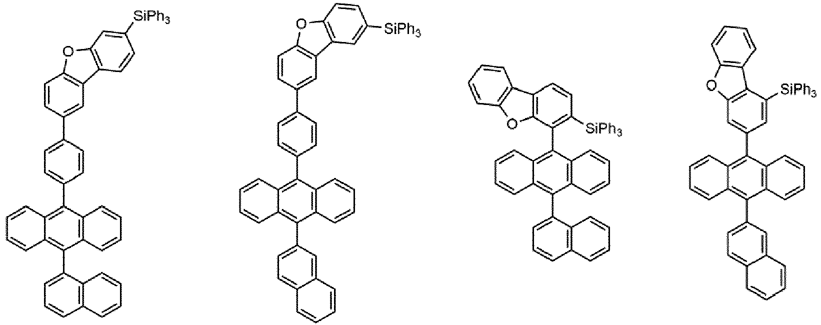

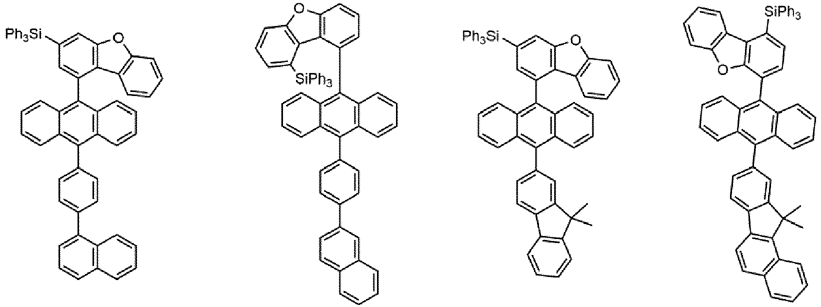

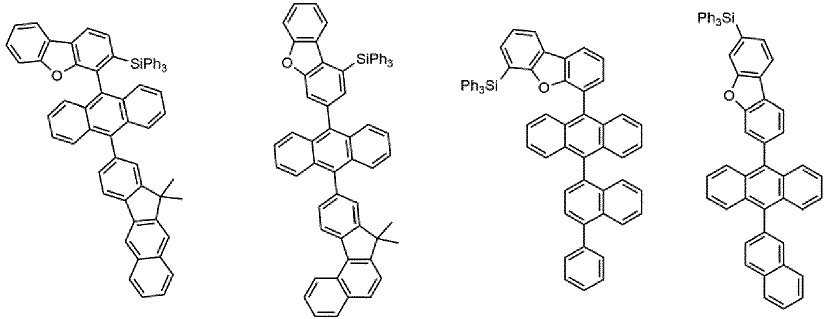

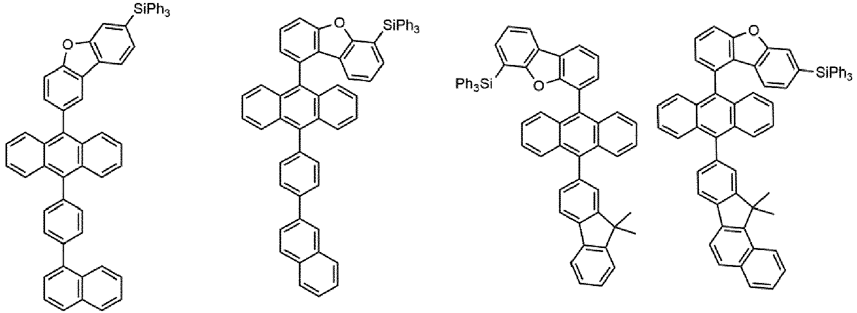

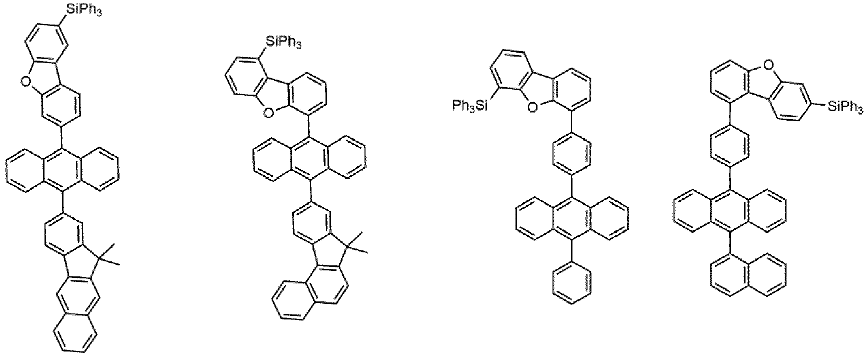

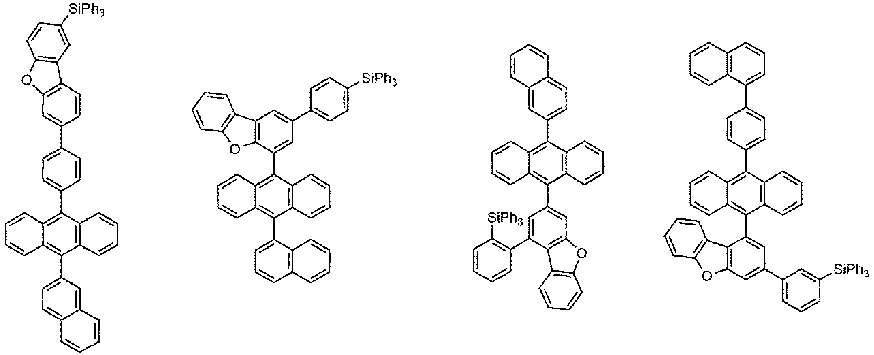

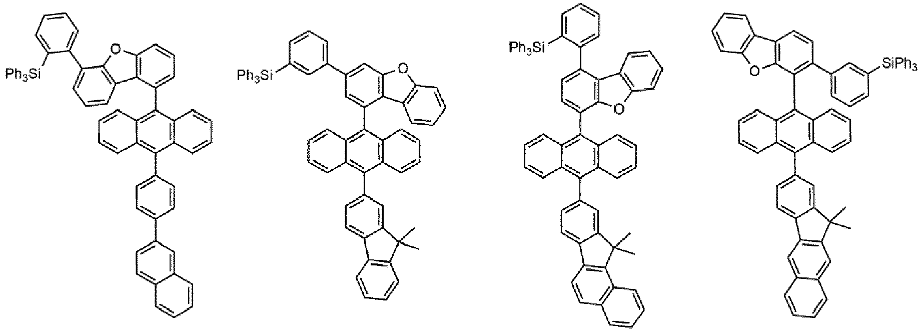

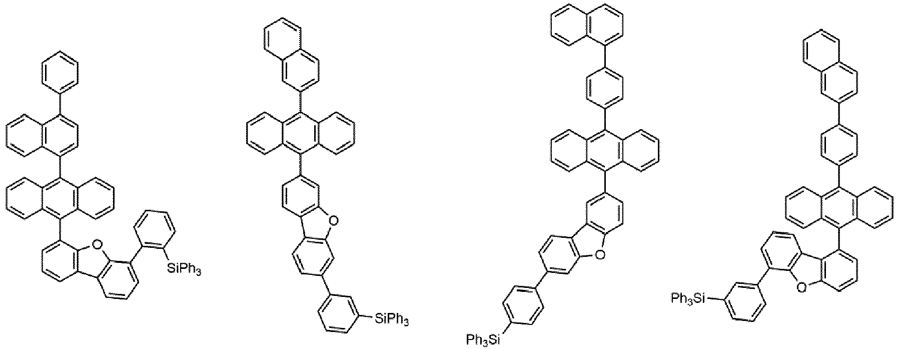

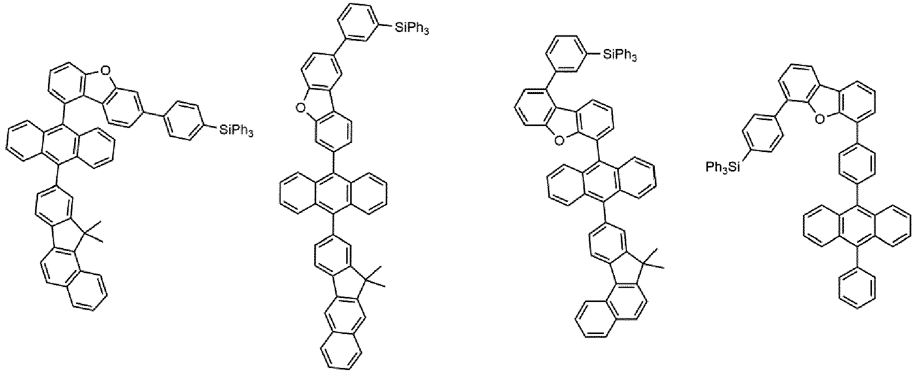

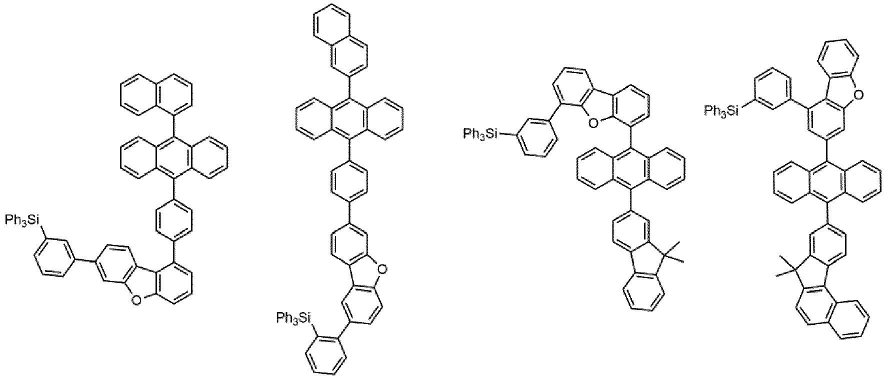

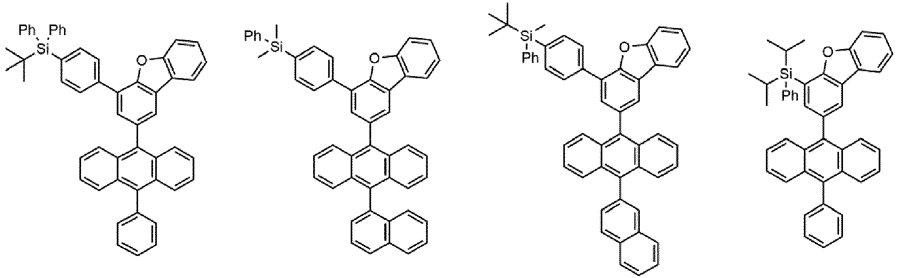

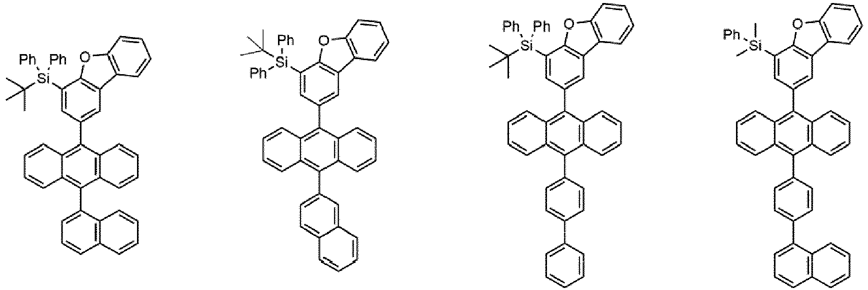

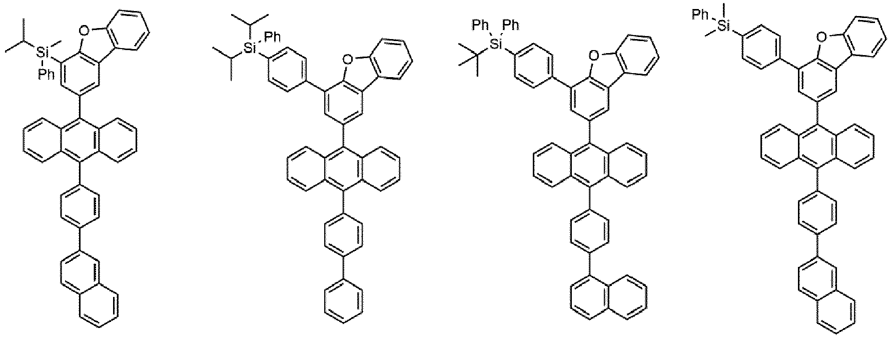

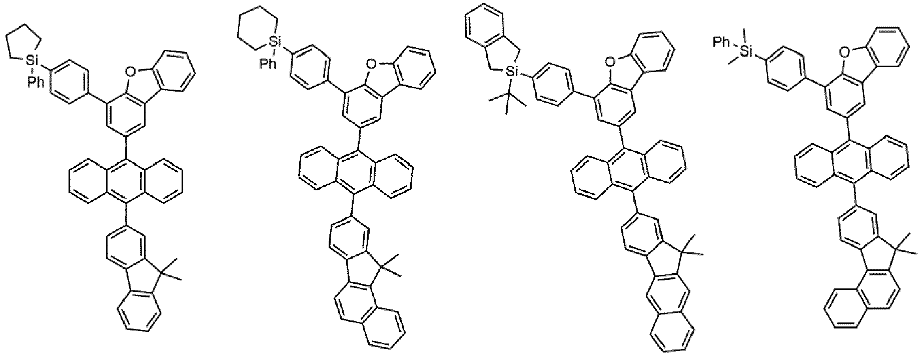

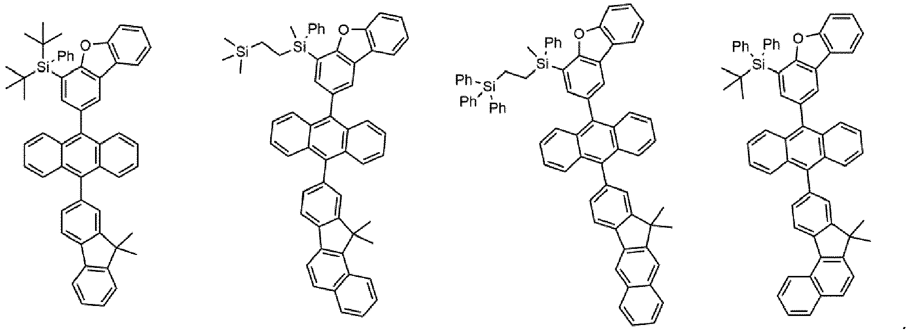

9. A coating composition comprising the compound according to any one of claims 1 to 8.

10. An organic light emitting device comprising: a first electrode; a second electrode provided opposite to the first electrode; and one or more organic material layers provided between the first electrode and the second electrode, wherein one or more of the organic material layers comprise the compound according to any one of claims 1 to 8.

11. The organic light emitting device according to claim 10, wherein the organic material layer comprising the compound is a light emitting layer.

## Patentansprüche

1. Verbindung mit der folgenden chemischen Formel 1: 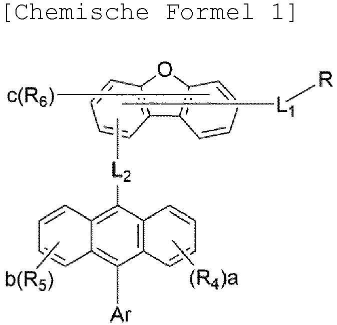 worin, in der chemischen Formel 1,
R Si(R₁)(R₂)(R₃) ist,
R₁ bis R₃ sind jeweils unabhängig Wasserstoff; ein substituiertes oder nicht-substituiertes C₁₋₆₀ Alkyl; ein substituiertes oder nicht-substituiertes C₆₋₆₀ Aryl; ein (Tri (C₁₋₆₀ alkyl)silyl)-(C₁₋₁₀) alkylen)-; oder ein (Tri(C₆₋₆₀ aryl) silyl) -(C₁₋₁₀ alkylen)-, oder R₁ und R₂ sind so miteinander verbunden, dass sie einen Ring bilden, vorausgesetzt dass der Fall, dass R₁ bis R₃ alle ein substituiertes oder nicht-substituiertes C₁₋₆₀ Alkyl sind, ausgenommen ist,
a bis c sind jeweils unabhängig eine ganze Zahl von 0 bis 4,
R₄ bis R₆ sind jeweils unabhängig Wasserstoff; Deuterium; ein Halogen; eine Cyanogruppe; ein substituiertes oder nicht-substituiertes C₁₋₆₀ Alkyl; ein substituiertes oder nicht-substituiertes C₁₋₆₀ Alkoxy; ein substituiertes oder nicht-substituiertes C₁₋₆₀ Thioalkyl; ein substituiertes oder nicht-substituiertes C₃₋₆₀ Cycloalkyl; ein substituiertes oder nicht-substituiertes C₆₋₆₀ Aryl; oder ein substituiertes oder nicht-substituiertes C₅₋₆₀ Heteroaryl, das ein oder mehr Heteroatome, ausgewählt aus der Gruppe bestehend aus N, O und S, enthält,
L₁ ist eine Einfachbindung; oder Phenylen,
L₂ ist eine Einfachbindung; oder ein substituiertes oder nicht-substituiertes C₆₋₆₀ Arylen, und
Ar ist ein substituiertes oder nicht-substituiertes C₆₋₆₀ Aryl; oder ein substituiertes oder nicht-substituiertes C₅₋₆₀ Heteroaryl, das ein oder mehr Heteroatome, ausgewählt aus der Gruppe bestehend aus N, O und S, enthält,
wobei der Ausdruck "substituiert" substituiert mit ein oder mehr Substituenten, ausgewählt aus der Gruppe bestehend aus Deuterium; einer Halogengruppe; einer Nitrilgruppe; einer Nitrogruppe; einer Hydroxygruppe; einer Carbonylgruppe; einer Estergruppe; einer Imidgruppe; einer Aminogruppe; einer Phosphinoxidgruppe; einer Alkoxygruppe; einer Aryloxygruppe; einer Alkylthioxygruppe; einer Arylthioxygruppe; einer Alkylsulfoxygruppe; einer Arylsulfoxygruppe; einer Silylgruppe; einer Borgruppe; einer Alkylgruppe; einer Cycloalkylgruppe; einer Alkenylgruppe; einer Arylgruppe; einer Aralkylgruppe; einer Aralkenylgruppe; einer Alkylarylgruppe; einer Alkylamingruppe; einer Aralkylamingruppe; einer Heteroarylamingruppe; einer Arylamingruppe; einer Arylphosphingruppe; und einer heterocyclischen Gruppe, die mindestens einen Vertreter aus N, O, und S Atomen enthält, oder mit einem Substituenten substituiert, an den zwei oder mehr Substituenten unter den Substituenten gebunden sind, bedeutet.

2. Verbindung gemäß Anspruch 1, wobei
die chemische Formel 1 durch die folgende chemische Formel 1-1 oder 1-2 dargestellt ist: 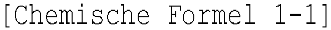 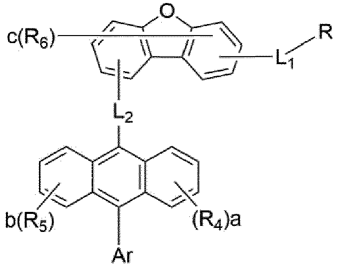 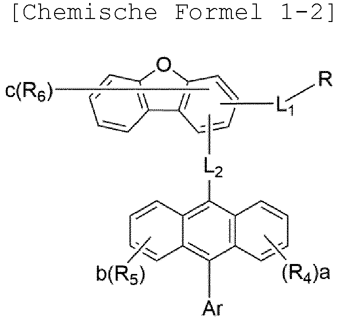 worin, in der chemischen Formel 1-1 oder 1-2, R, a bis c, R₄ bis R₆, L₁, L₂, und Ar wie in Anspruch 1 definiert sind.

3. Verbindung gemäß Anspruch 1, wobei
a bis c alle 0 sind.

4. Verbindung gemäß Anspruch 1, wobei
R₁ bis R₃ jeweils unabhängig Methyl; i-Propyl; t-Butyl; Phenyl; Trimethylsilyl-ethylen; oder Triphenylsilyl-ethylen sind, oder R₁ und R₂ sind so verbunden, dass sie einen Cyclopentyl-, Cyclohexyl-, oder 2,3-dihydro-1H-indenyl-Ring bilden.

5. Verbindung gemäß Anspruch 1, wobei
mindestens einer aus R₁ bis R₃ Phenyl ist.

6. Verbindung gemäß Anspruch 1, wobei
L₂ eine Einfachbindung oder Phenylen ist.

7. Verbindung gemäß Anspruch 1, wobei
Ar mindestens einer ist, ausgewählt aus der Gruppe bestehend aus den folgenden Vertretern:

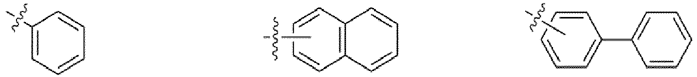

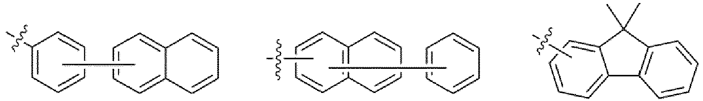

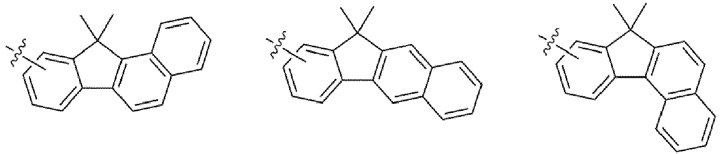

8. Verbindung gemäß Anspruch 1, wobei
die Verbindung mit der chemischen Formel 1 ausgewählt ist aus der Gruppe bestehend aus den folgenden Vertretern:

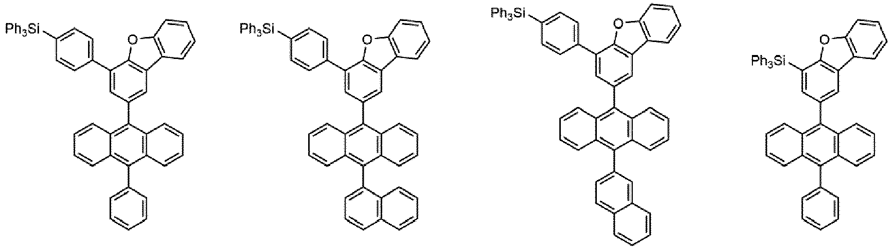

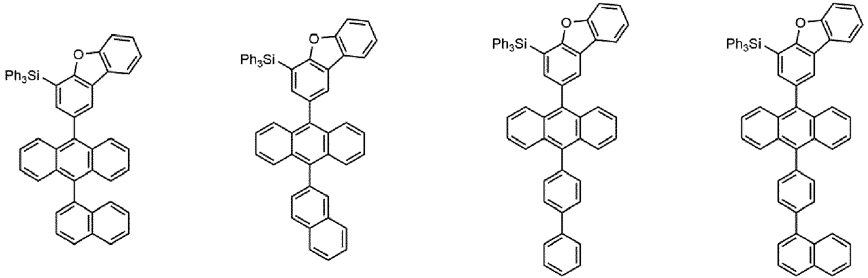

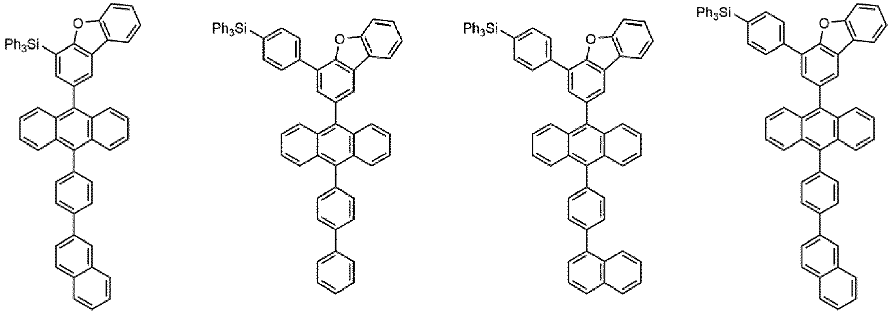

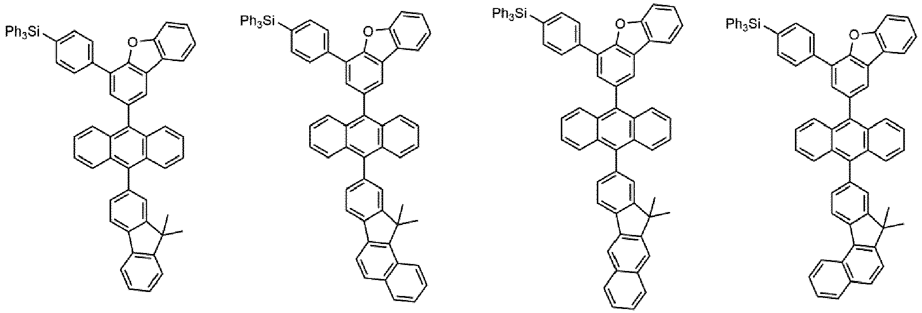

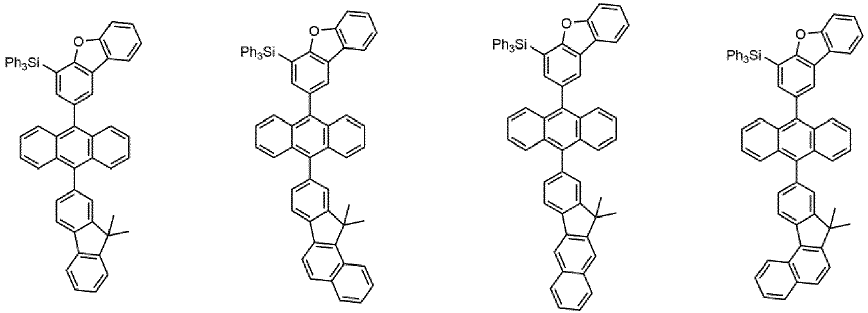

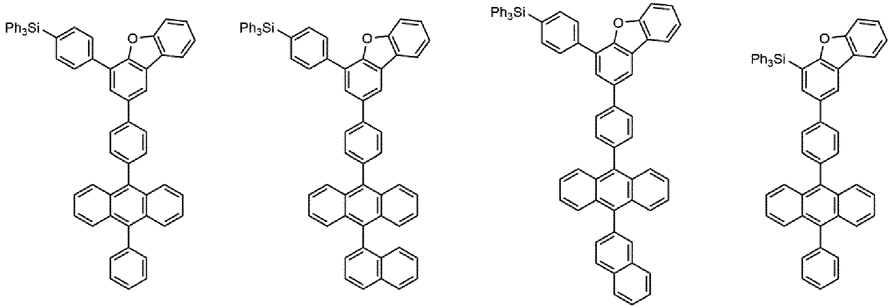

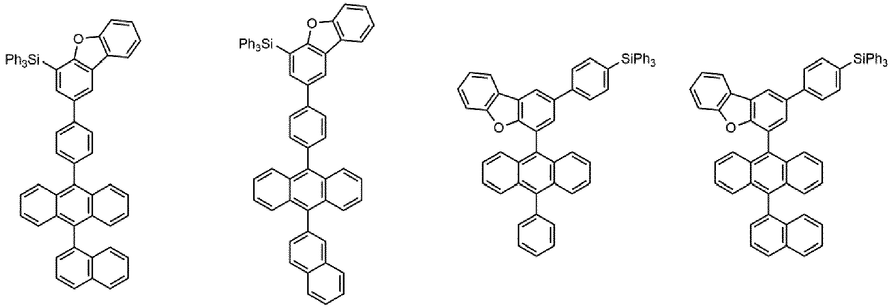

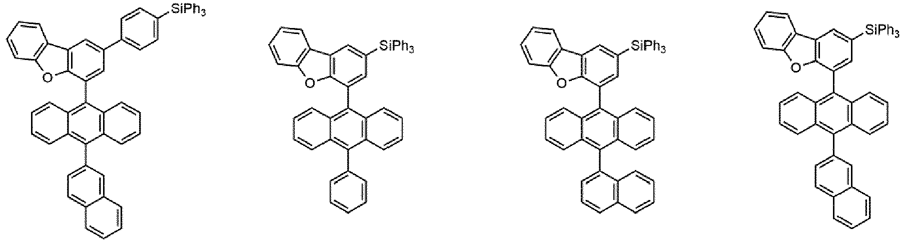

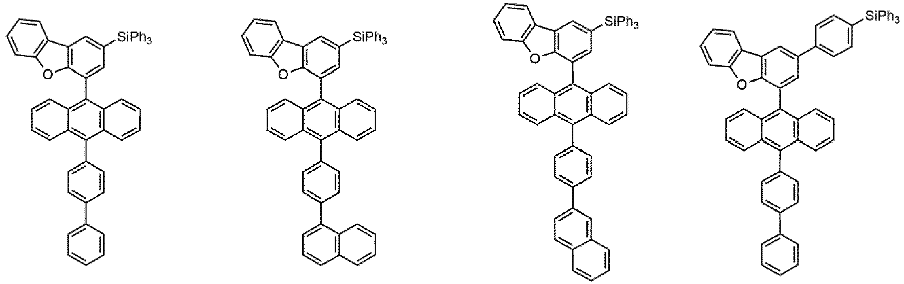

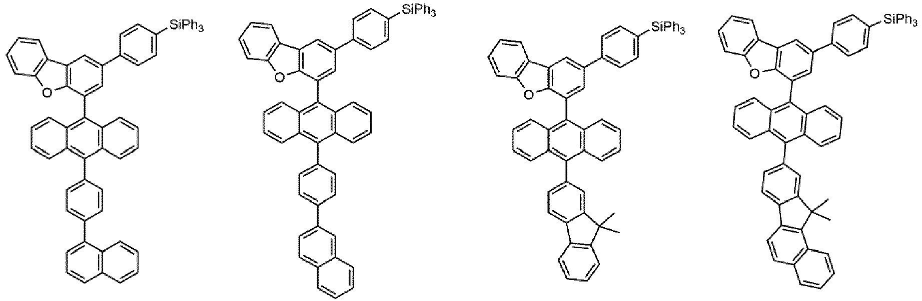

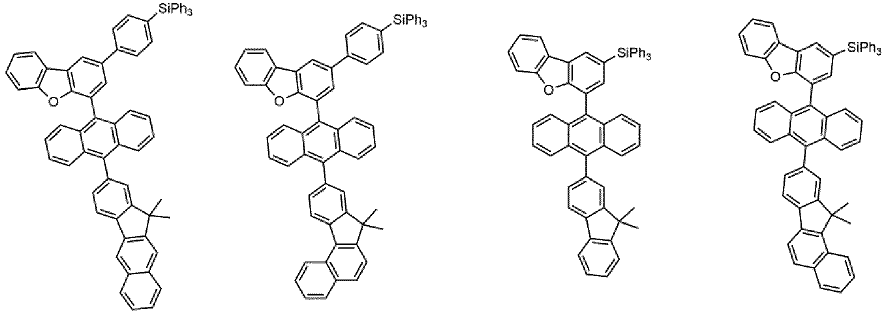

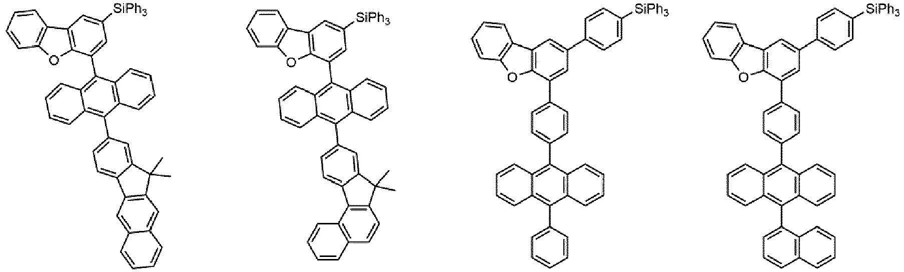

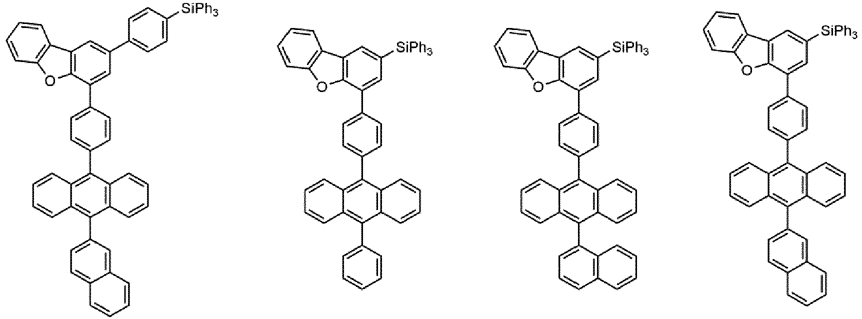

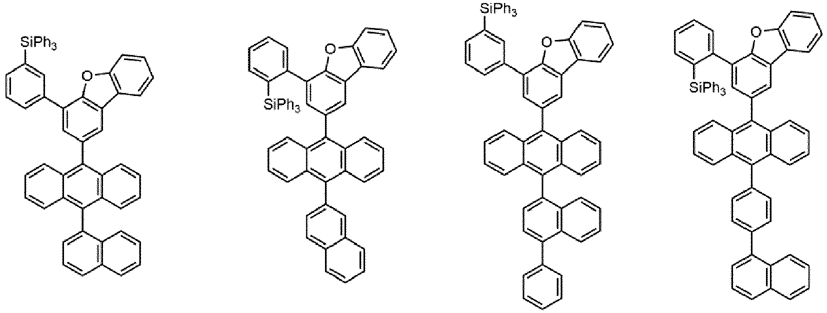

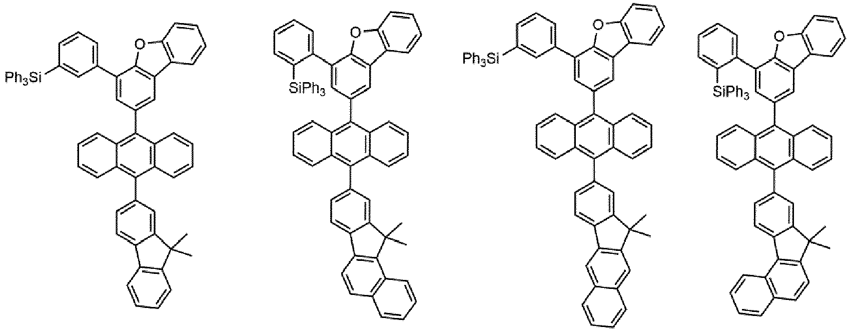

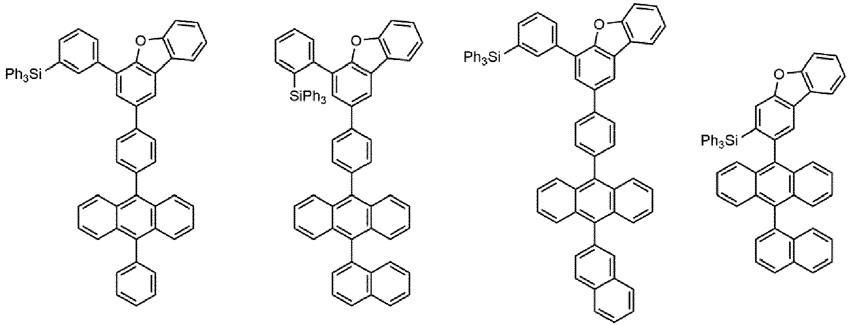

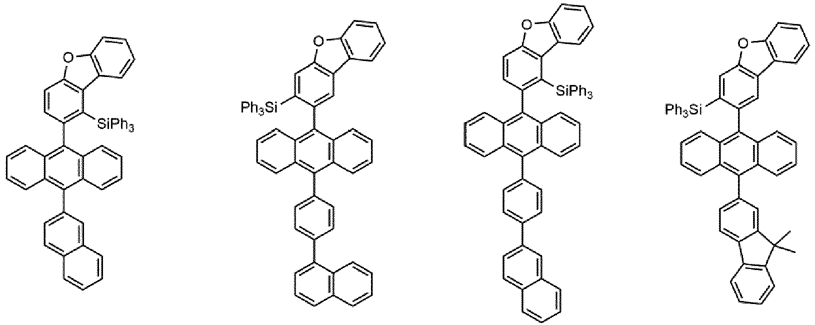

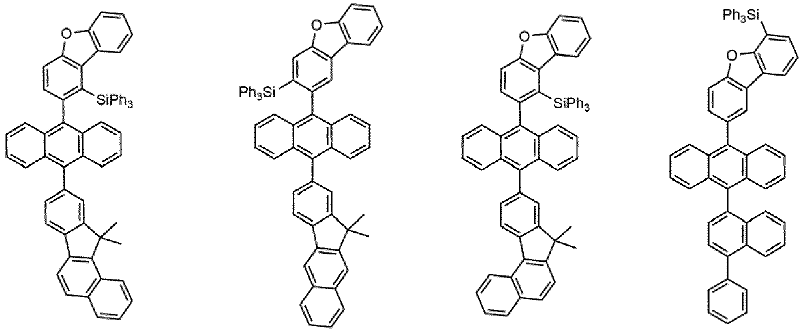

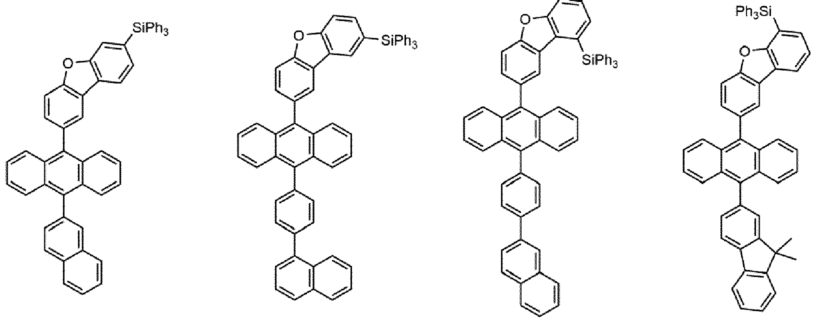

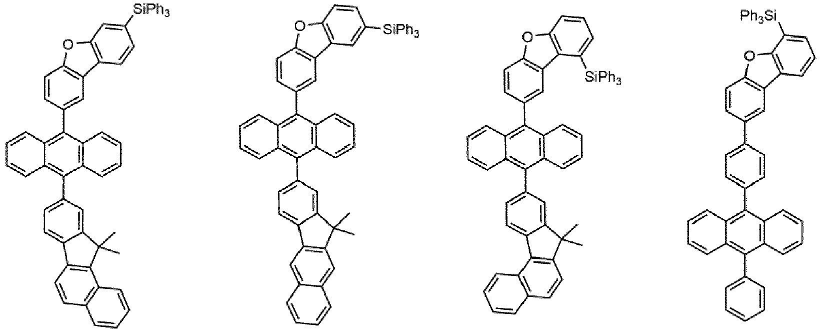

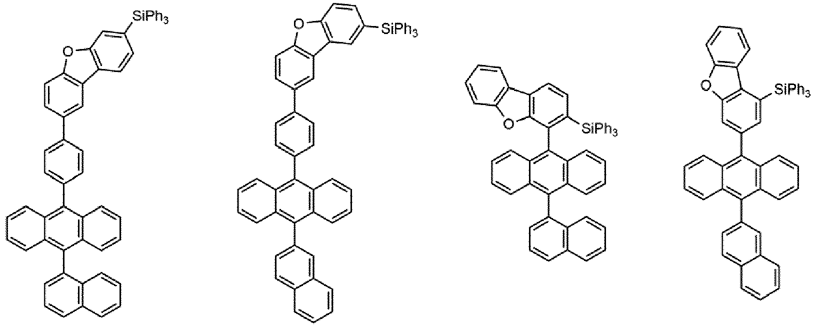

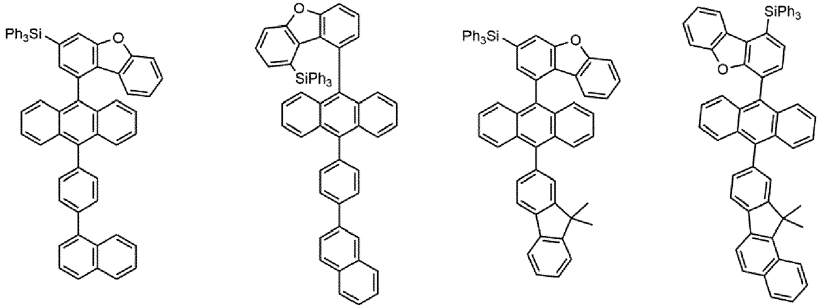

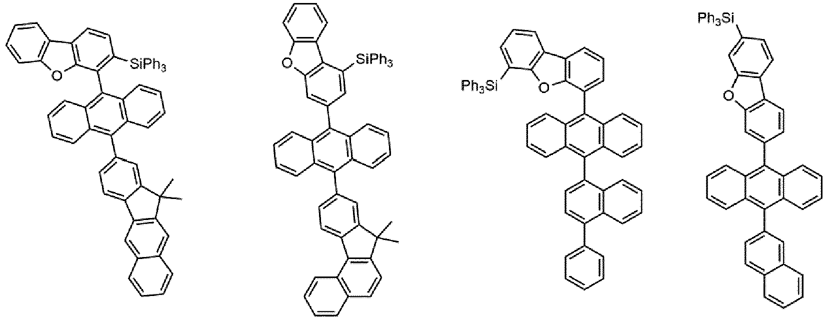

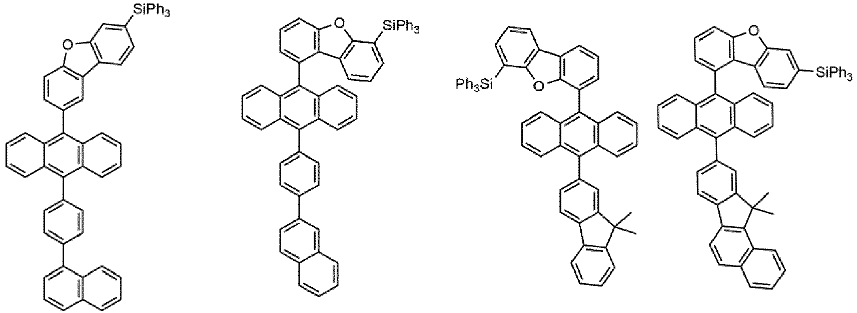

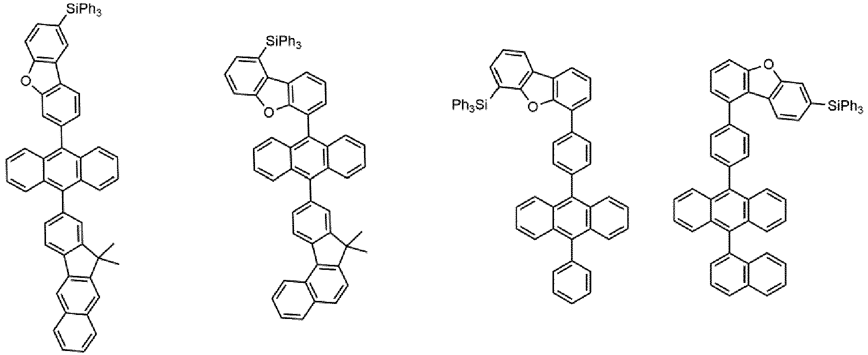

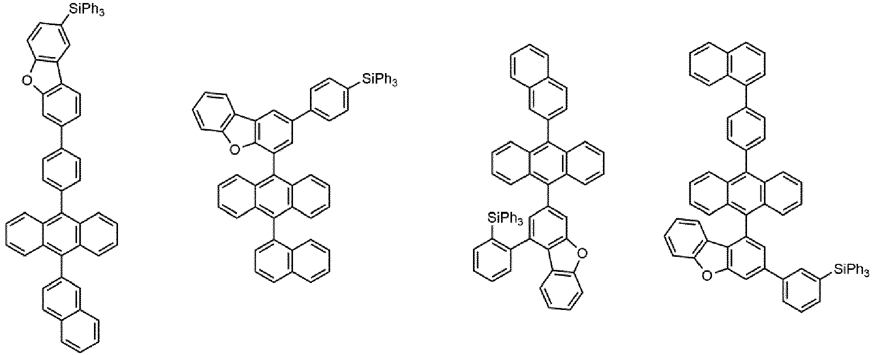

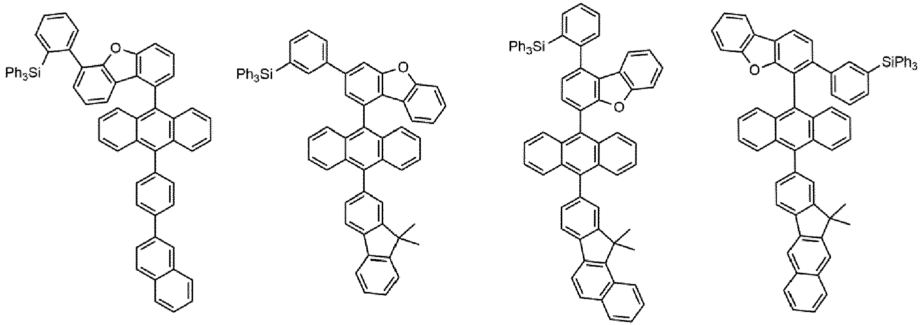

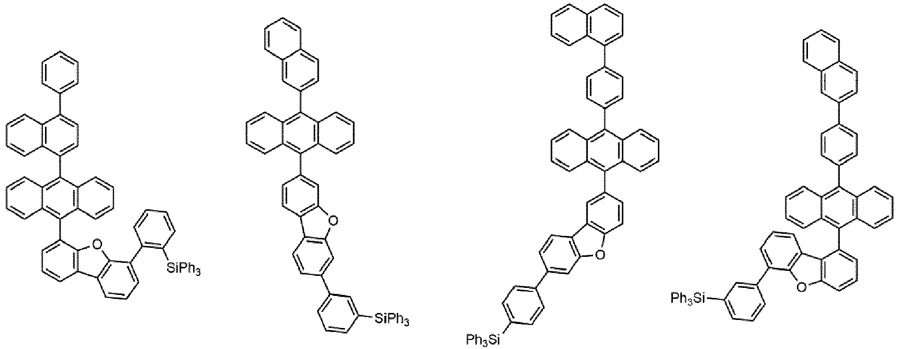

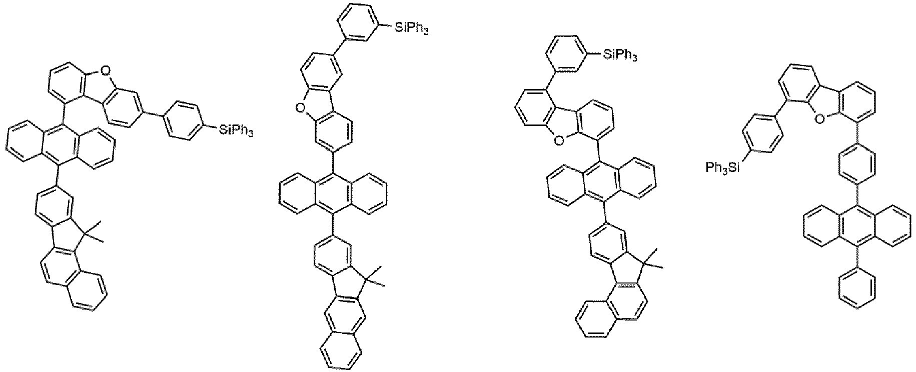

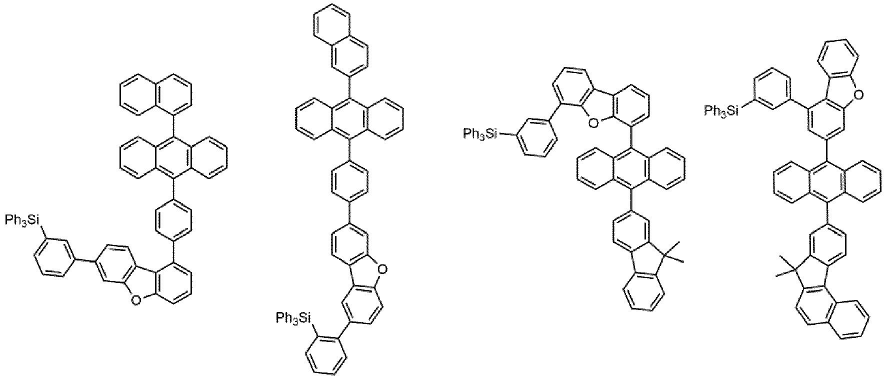

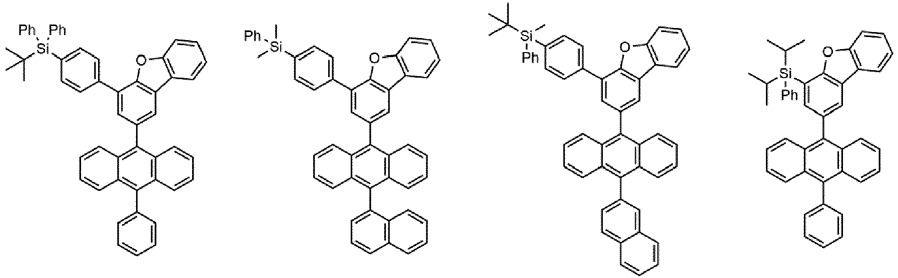

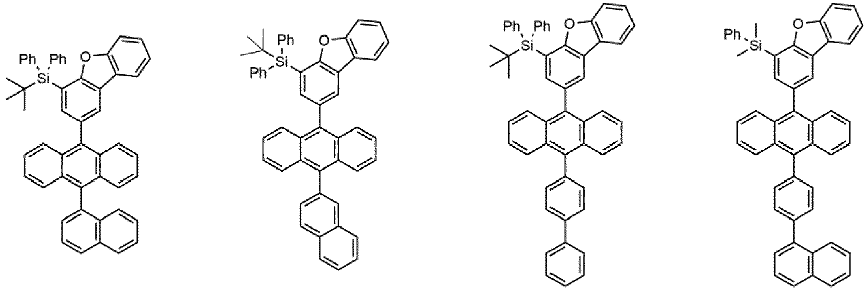

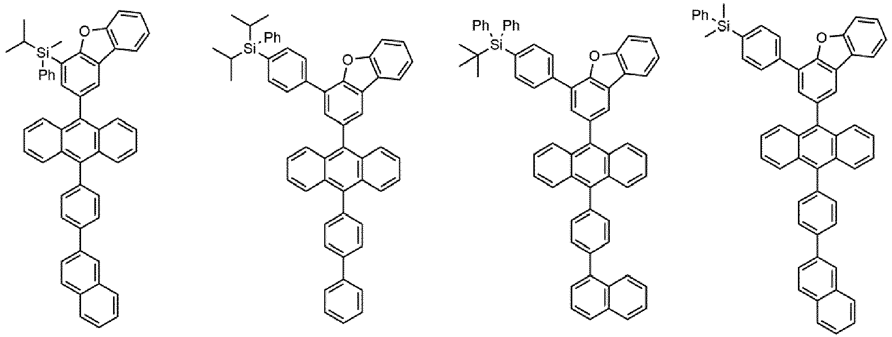

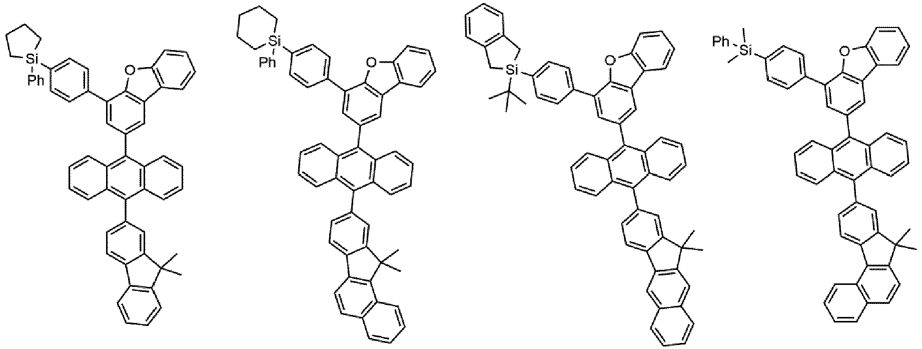

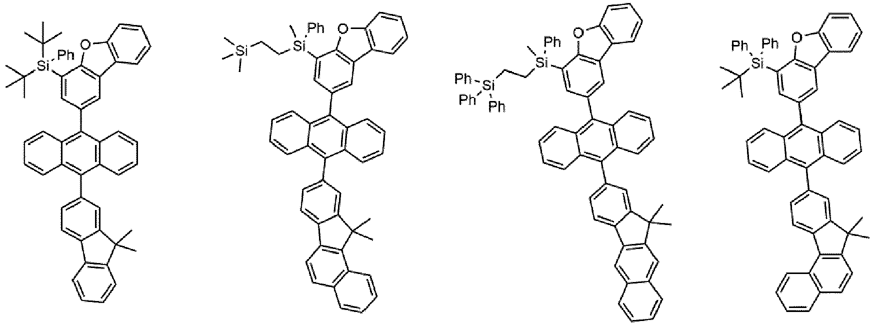

9. Beschichtungszusammensetzung, die die Verbindung gemäß mindestens einem der Ansprüche 1 bis 8 umfasst.

10. Organische lichtemittierende Vorrichtung, umfassend: eine erste Elektrode; eine zweite Elektrode, die gegenüber der ersten Elektrode angeordnet ist; und ein oder mehr organische Materialschichten, die zwischen der ersten Elektrode und der zweiten Elektrode angeordnet sind, wobei ein oder mehr der organischen Materialschichten die Verbindung gemäß mindestens einem der Ansprüche 1 bis 8 umfassen.

11. Organische lichtemittierende Vorrichtung gemäß Anspruch 10, wobei die organische Materialschicht, die die Verbindung umfasst, eine lichtemittierende Schicht ist.

## Revendications

1. Composé représenté par la formule chimique 1 suivante : 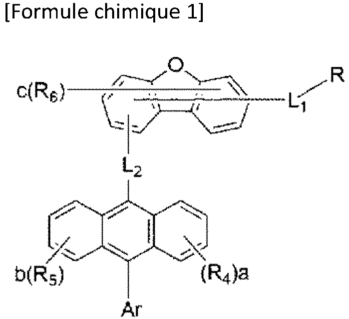 dans lequel, dans la formule chimique 1,
R est Si(R₁)(R₂)(R₃),
R₁ à R₃ sont chacun indépendamment l'hydrogène ; un alkyle en C₁₋₆₀ substitué ou non substitué; un aryle en C₆₋₆₀ substitué ou non substitué; un (tri(C₁₋₆₀ alkyl)silyl)-(C₁₋₁₀ alkylène)-; ou un (tri(C₆₋₆₀ aryl)silyl)-(C₁₋₁₀ alkylène)-, ou R₁ et R₂ sont liés ensemble pour former un cycle, à la condition que le cas dans lequel R₁ à R₃ sont tous un alkyle en C₁₋₆₀ substitué ou non substitué soit exclu,
a à c sont chacun indépendamment un nombre entier de 0 à 4,
R₄ à R₆ sont chacun indépendamment l'hydrogène ; le deutérium ; un halogène ; un groupe cyano ; un alkyle en C₁₋₆₀ substitué ou non substitué ; un alcoxy en C₁₋₆₀ substitué ou non substitué ; un thioalkyle en C₁₋₆₀ substitué ou non substitué ; un cycloalkyle en C₃₋₆₀ substitué ou non substitué ; un aryle en C₆₋₆₀ substitué ou non substitué ; ou un hétéroaryle en C₅₋₆₀ substitué ou non substitué contenant un ou plusieurs hétéroatomes choisis dans le groupe constitué par N, 0 et S,
L₁ est une liaison simple ; ou le phénylène,
L₂ est une liaison simple ; ou un arylène en C₆₋₆₀substitué ou non substitué, et
Ar est un aryle en C₆₋₆₀ substitué ou non substitué ; ou un hétéroaryle en C₅₋₆₀ substitué ou non substitué contenant un ou plusieurs hétéroatomes choisis dans le groupe constitué par N, 0 et S,
dans lequel le terme « substitué » signifie substitué par un ou plusieurs substituants choisis dans le groupe constitué par le deutérium ; un groupe halogène ; un groupe nitrile ; un groupe nitro ; un groupe hydroxy ; un groupe carbonyle ; un groupe ester ; un groupe imide ; un groupe amino ; un groupe d'oxyde de phosphine ; un groupe alcoxy ; un groupe aryloxy ; un groupe alkylthioxy ; un groupe arylthioxy ; un groupe alkylsulfoxy ; un groupe arylsulfoxy ; un groupe silyle ; un groupe bore ; un groupe alkyle ; un groupe cycloalkyle ; un groupe alcényle ; un groupe aryle ; un groupe aralkyle ; un groupe aralcényle ; un groupe alkylaryle ; un groupe alkylamine ; un groupe aralkylamine ; un groupe hétéroarylamine ; un groupe arylamine ; un groupe arylphosphine ; et un groupe hétérocyclique contenant au moins un des atomes N, 0 et S, ou étant substitué par un substituant auquel deux substituants ou plus sont liés parmi lesdits substituants.

2. Composé selon la revendication 1, dans lequel
la formule chimique 1 est représentée par la formule chimique 1-1 ou 1-2 suivante : 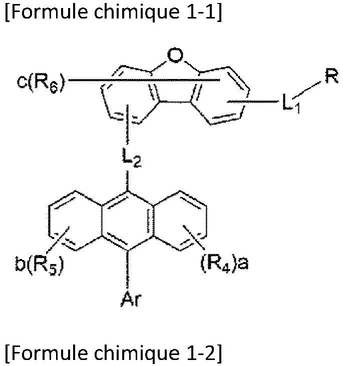 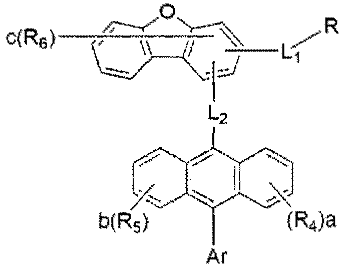 dans lequel, dans les formules chimiques 1-1 ou 1-2,
R, a à c, R₄ à R₆, L₁, L₂, et Ar sont tels que définis dans la revendication 1.

3. Composé selon la revendication 1, dans lequel a à c sont tous 0.

4. Composé selon la revendication 1, dans lequel
R₁ à R₃ sont chacun indépendamment le méthyle ; l'i-propyle; le t-butyle ; le phényle ; le triméthylsilyl-éthylène ; ou le triphénylsilyl-éthylène, ou R₁ et R₂ sont liés ensemble pour former un cycle cyclopentyle, cyclohexyle, ou 2,3-dihydro-1 H-indényle.

5. Composé selon la revendication 1, dans lequel
au moins un parmi R₁ à R₃ est le phényle.

6. Composé selon la revendication 1, dans lequel
L₂ est une liaison simple ou le phénylène.

7. Composé selon la revendication 1, dans lequel
Ar est l'un quelconque choisi dans le groupe constitué par les suivants :

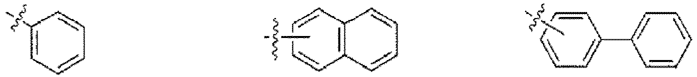

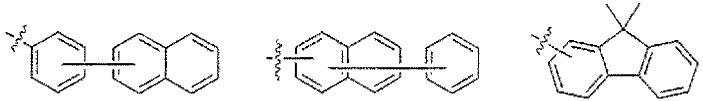

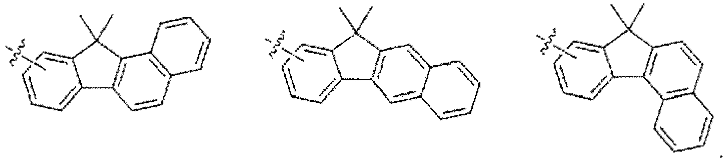

8. Composé selon la revendication 1, dans lequel
le composé représenté par la formule chimique 1 est choisi dans le groupe constitué par les composés suivants :

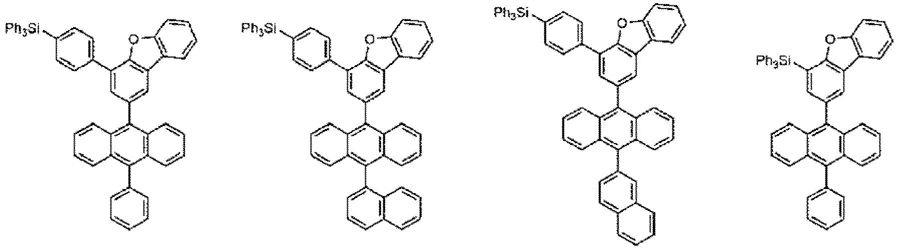

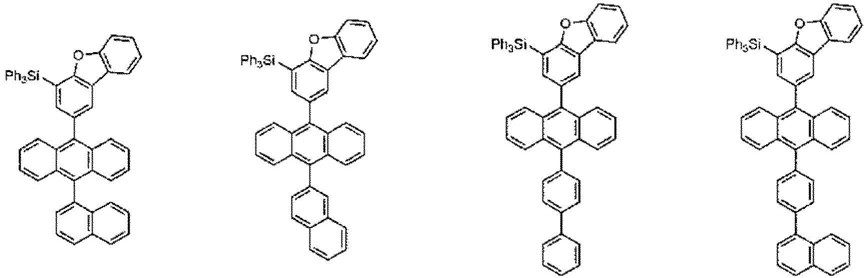

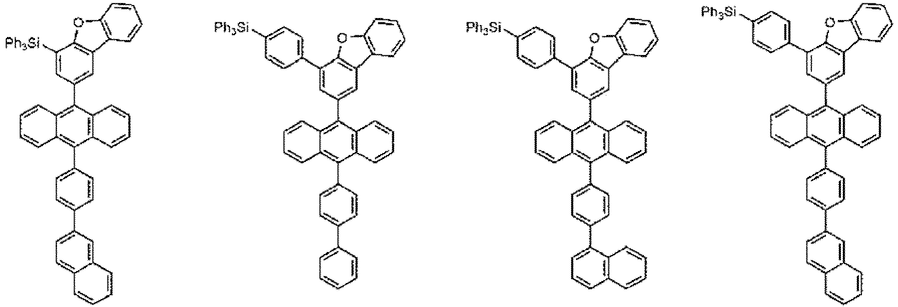

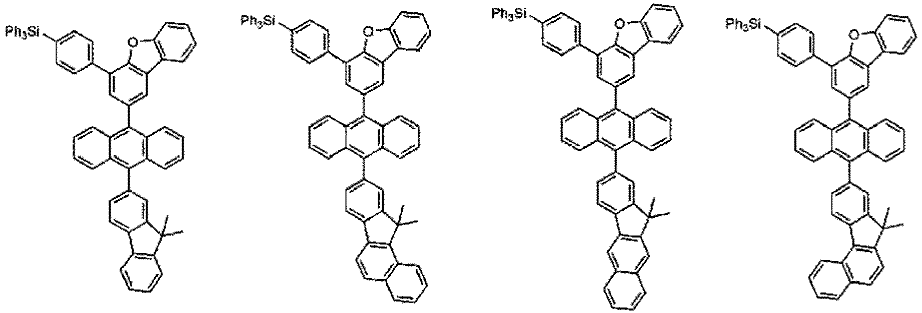

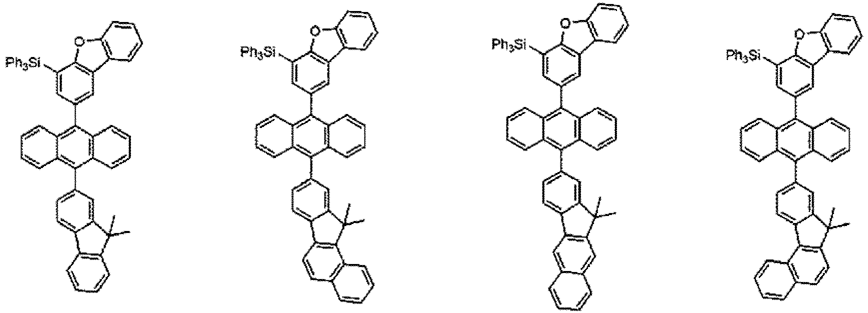

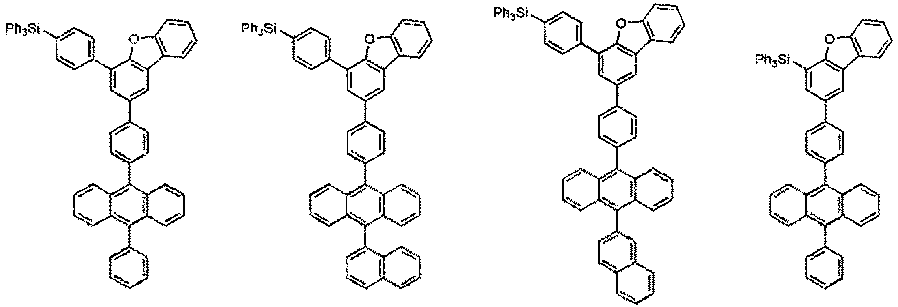

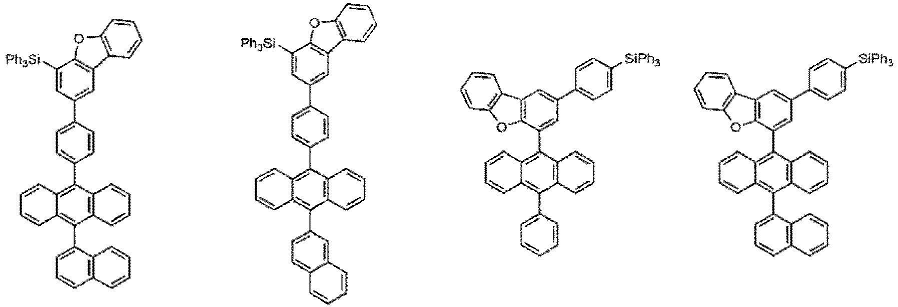

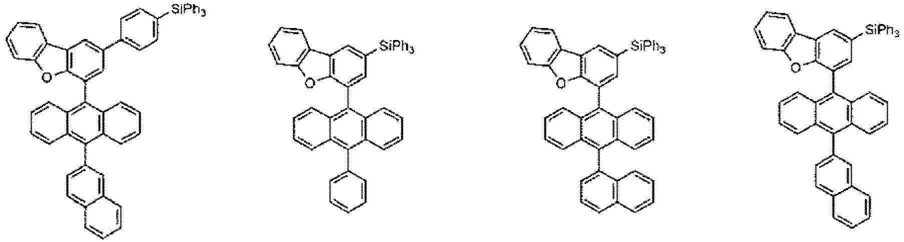

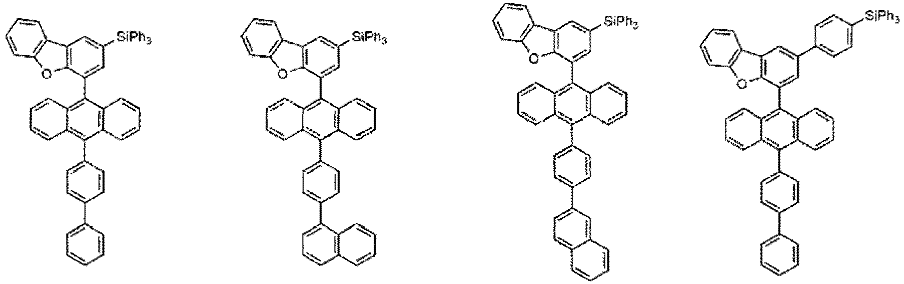

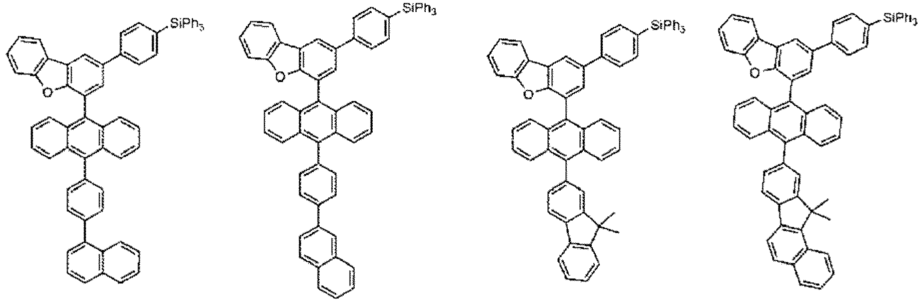

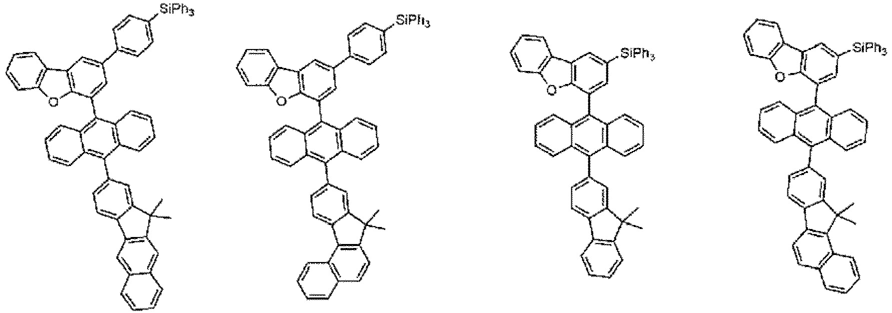

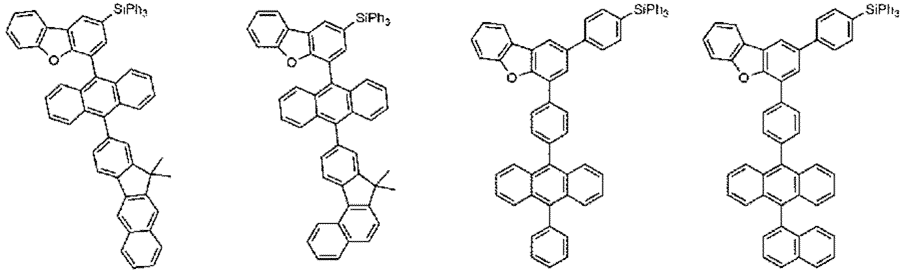

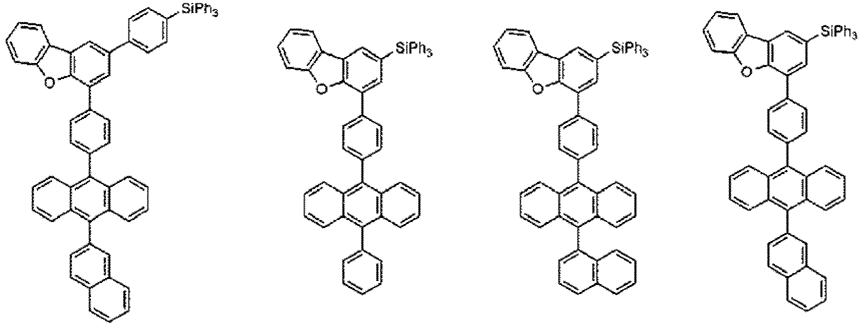

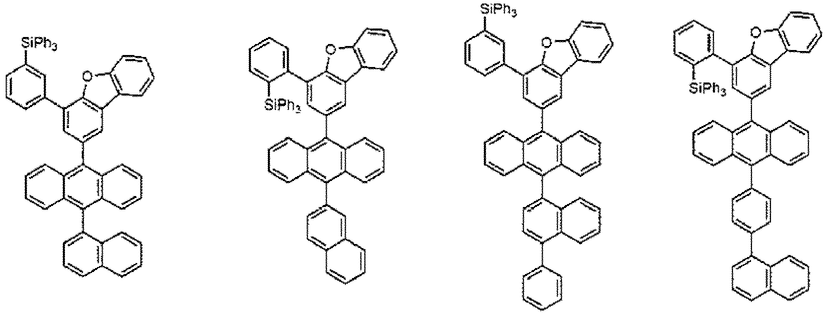

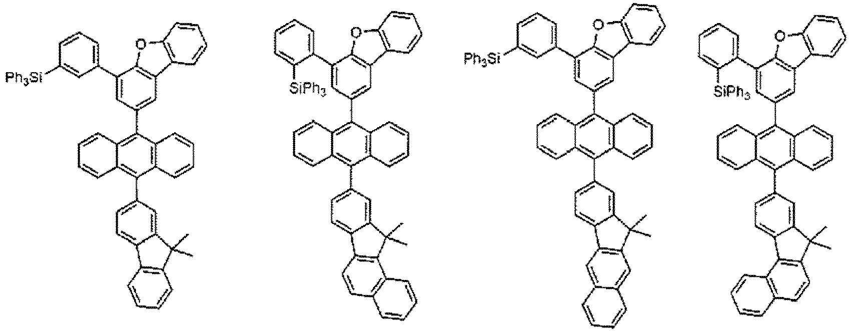

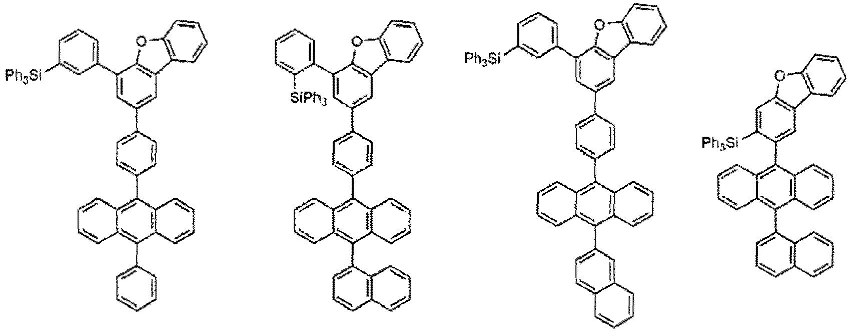

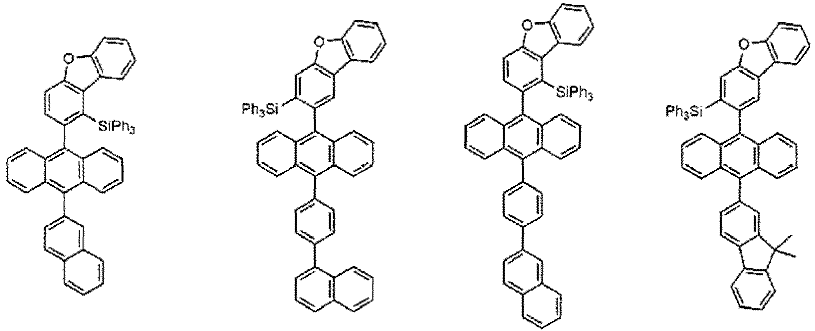

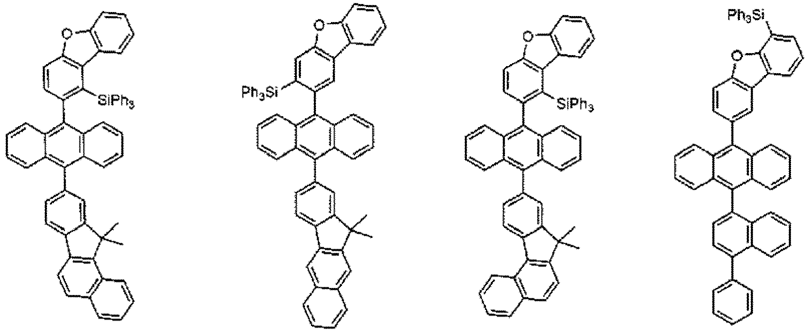

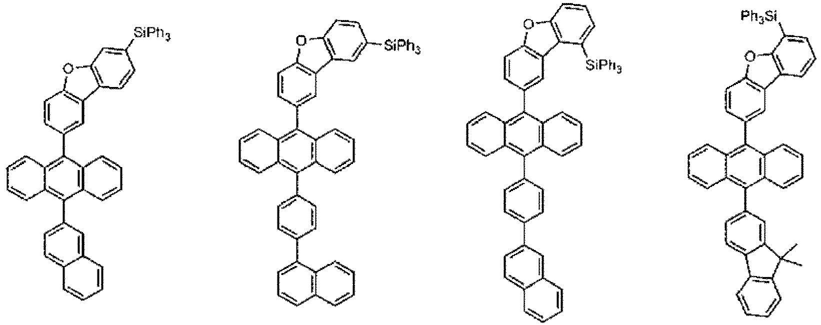

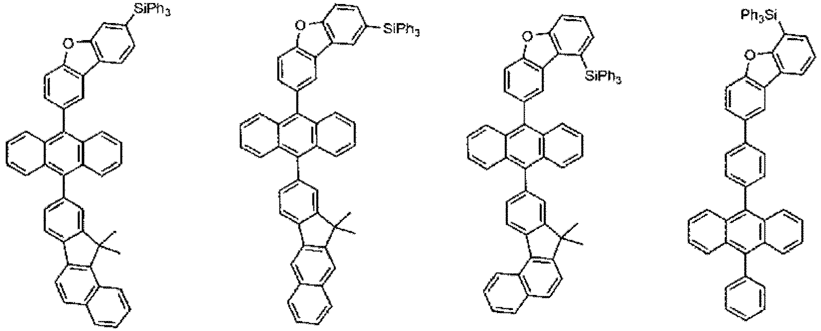

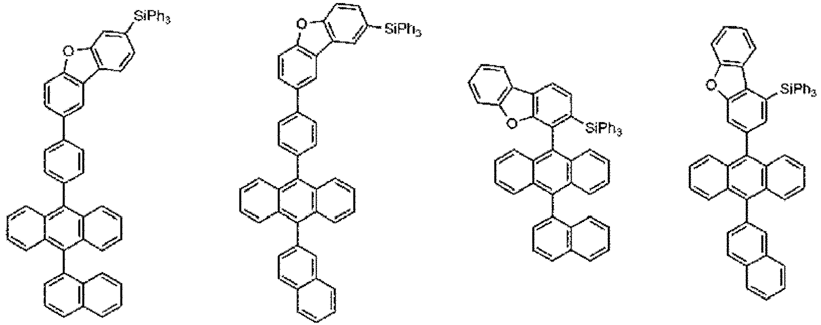

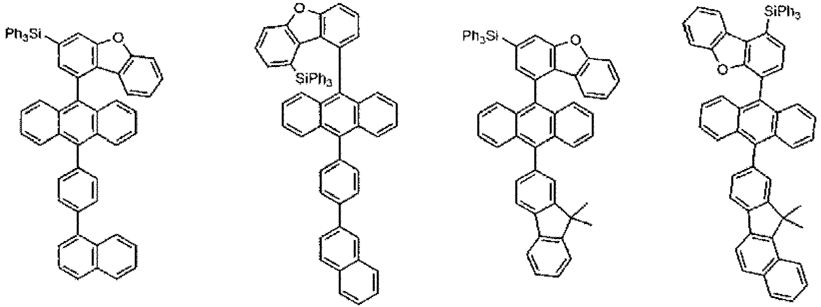

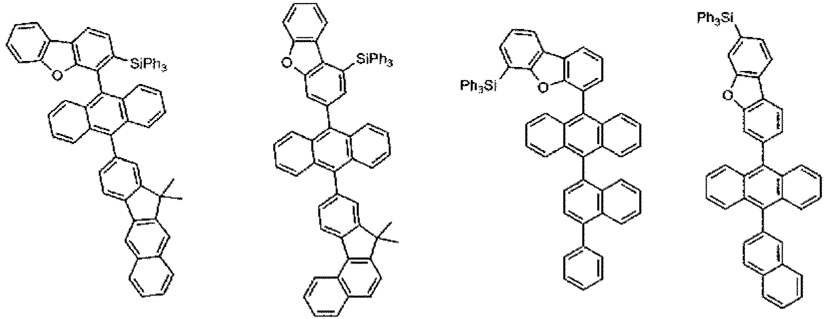

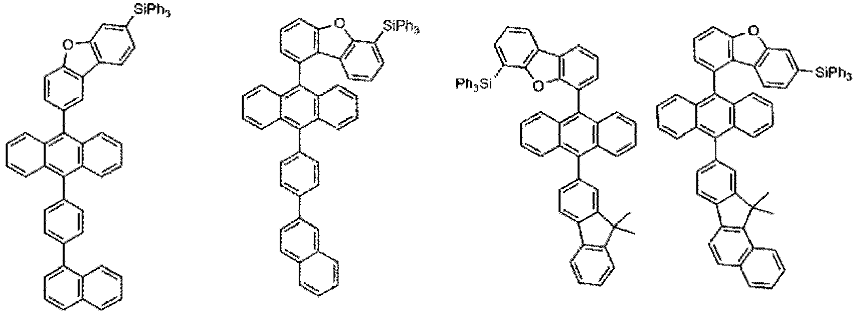

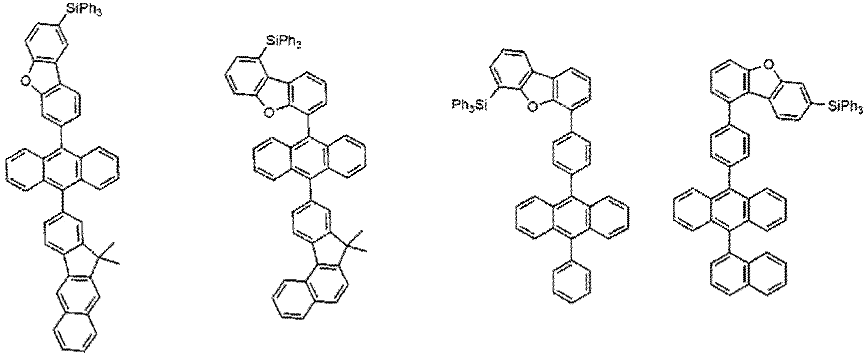

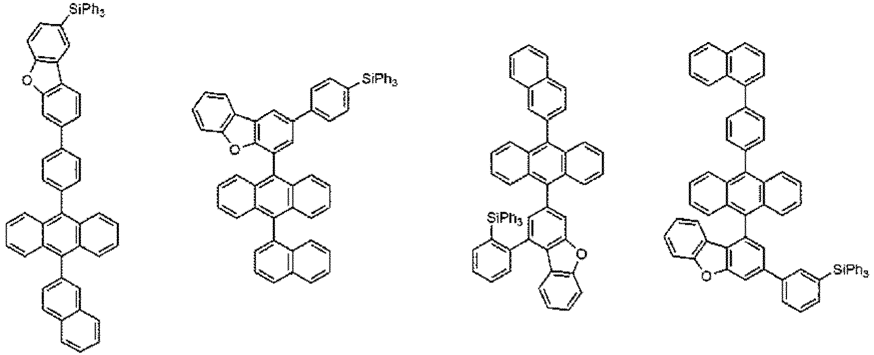

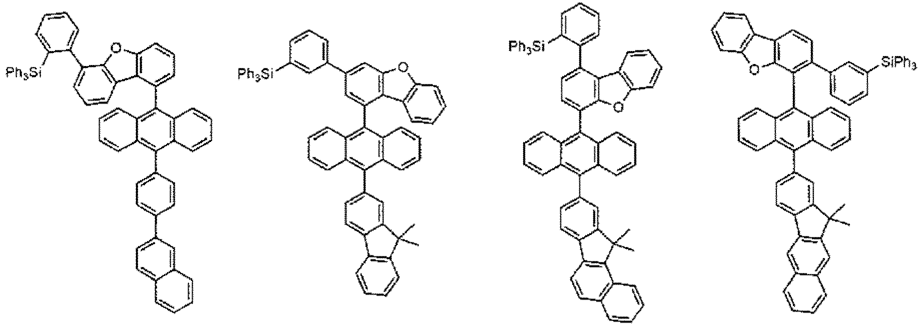

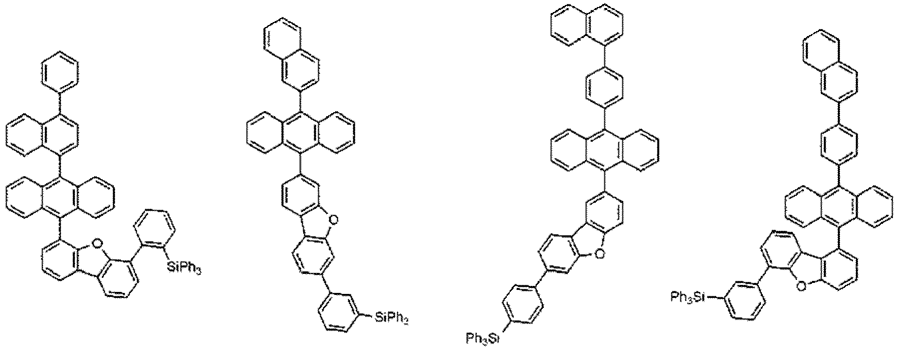

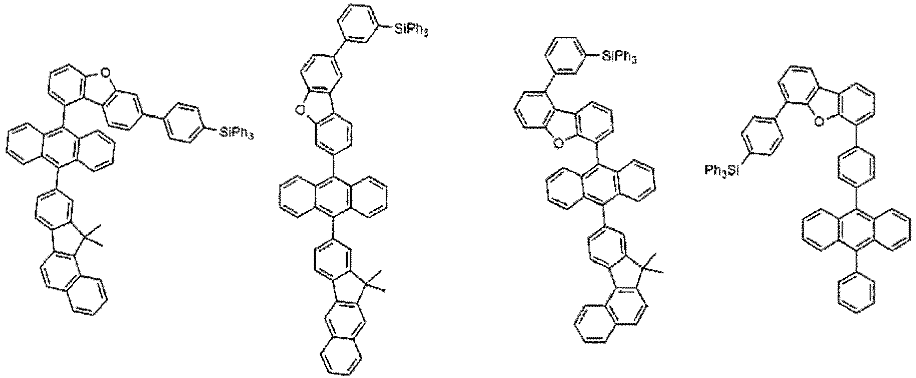

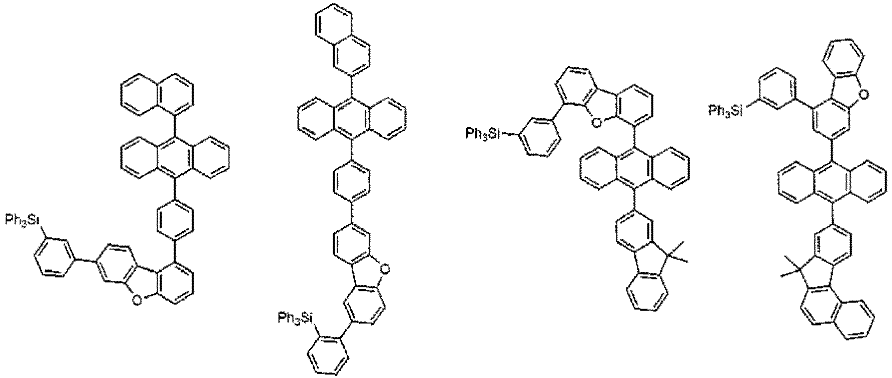

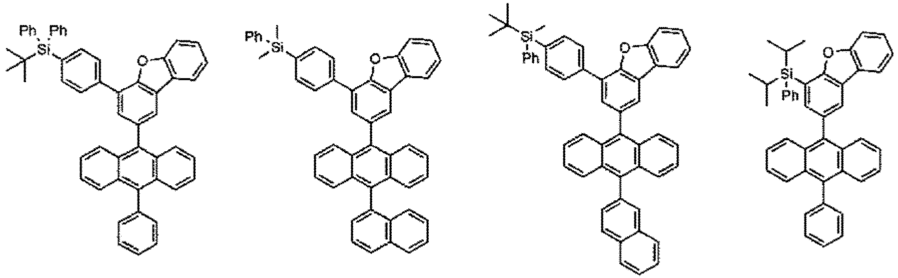

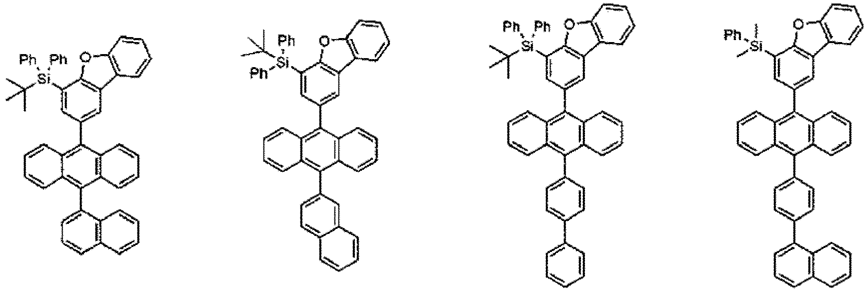

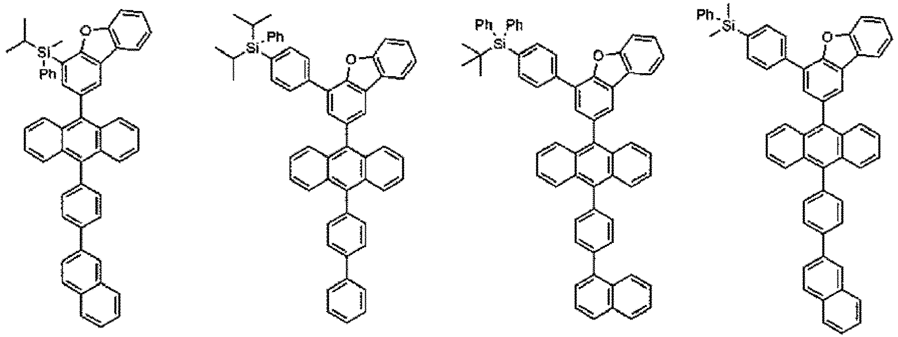

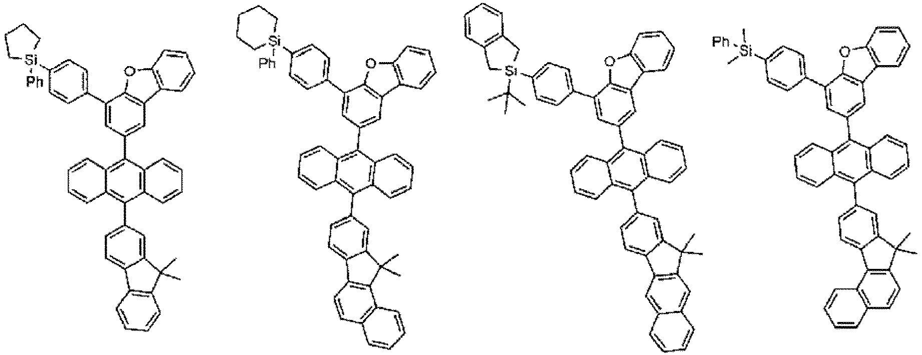

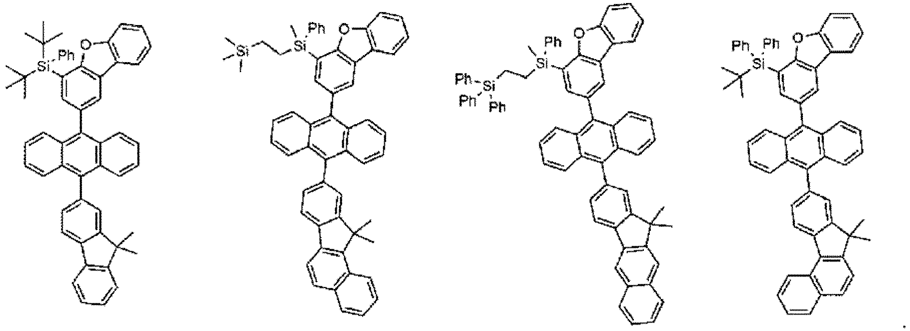

9. Composition de revêtement comprenant le composé selon l'une quelconque des revendications 1 à 8.

10. Dispositif électroluminescent organique comprenant : une première électrode ; une seconde électrode disposée à l'opposé de la première électrode ; et une ou plusieurs couches de matériau organique disposées entre la première électrode et la seconde électrode, dans lequel une ou plusieurs couches des couches de matériau organique comprennent le composé selon l'une quelconque des revendications 1 à 8.

11. Dispositif électroluminescent organique selon la revendication 10, dans lequel la couche de matériau organique comprenant le composé est une couche électroluminescente.
